# EUROPEAN PATENT SPECIFICATION

(11) **EP 4 248 998 B1**
(45) Date of publication and mention of the grant of the patent: **08.07.2026**
(21) Application number: 22860604.2
(22) Date of filing: 25.08.2022
(51) Int. Cl.: A61K 38/17, A61K 45/00, A61K 49/00, A61K 38/20, A61P 39/00, G01N 33/68, C07K 16/00, A61K 45/06

(54) **AGONISTS AND ANTAGONISTS OF IL-27 SIGNALLING FOR A THERAPEUTIC USE IN CONTROLLING BODY TEMPERATURE**
AGONISTEN UND ANTAGONISTEN DER DES IL-27 SIGNALWEGS ZUR THERAPEUTISCHEN VERWENDUNG BEI DER REGULIERUNG DER KÖRPERTEMPERATUR
AGONISTES ET ANTAGONISTES DE LA SIGNALISATION DE L'IL-27 POUR UNE UTILISATION THÉRAPEUTIQUE DANS LE CONTRÔLE DE LA TEMPÉRATURE CORPORELLE

(30) Priority: 26.08.2021 CN 202110986914
(43) Date of publication of application: 27.09.2023
(73) Proprietor: Guangdong Jiantebo Biotechnology Co., Ltd., Zhongshan, Guangdong 528400 (CN)
(72) Inventor: YIN, Zhinan, Zhongshan, Guangdong 528400 (CN); WANG, Qian, Zhongshan, Guangdong 528400 (CN); YANG, Hengwen, Zhongshan, Guangdong 528400 (CN)
(74) Representative: Office Freylinger
(86) International application number: PCT/CN2022/114928
(87) International publication number: WO 2023/025258

(56) References cited:
- EP-A1- 2 700 409
- WO-A1-2011/133931
- WO-A1-2016/073704
- WO-A1-2018/075740
- WO-A2-2010/118243
- CN-A- 101 039 959
- CN-A- 105 854 019
- CN-A- 107 661 497
- CN-A- 113 694 201
- JP-A- 2015 047 157
- WANG QIAN ET AL: "IL-27 signalling promotes adipocyte thermogenesis and energy expenditure", NATURE,, vol. 600, no. 7888, 24 November 2021 (2021-11-24), pages 314 - 318, XP037637888, DOI: 10.1038/S41586-021-04127-5
- ZHANG HENG, LI QINGJIE, TENG YUXIN, LIN YUBI, LI SHAOJIAN, QIN TINGFENG, CHEN LINXI, HUANG JIANA, ZHAI HENING, YU QUAN, XU GEYANG: "Interleukin-27 decreases ghrelin production through signal transducer and activator of transcription 3—mechanistic target of rapamycin signaling", ACTA PHARMACEUTICA SINICA B, vol. 10, no. 5, 1 May 2020 (2020-05-01), pages 837 - 849, XP093040020, ISSN: 2211-3835, DOI: 10.1016/j.apsb.2019.12.018
- CHUN-MEI WANG, CHENG TING-TING, JIAN-WEN △ BAI, LIN MIN-JIA, LIU XIAN-DONG: "Value of Combination Detection of Interleukin-27 and Procalcitonin as a Sepsis Diagnostic Biomarker in Critically Ill Adults*", PROGRESS IN MODERN BIOMEDICINE, vol. 15, no. 35, 20 December 2015 (2015-12-20), pages 6956 - 6960, XP093040016, DOI: 10.13241/j.cnki.pmb.2015.35.042

## Description

### Technical Field

The present disclosure relates to the field of biotechnology, and particularly relates to a composition, a method and a use, and particularly to a composition and a method for controlling heat generation within an organism, and a use.

### Background Art

Heat generation of brown and beige fat plays a key role in a development of maintenance of a body temperature. Although orientation and activation of the brown/beige fat are known, diversity and abundance of immune factors in adipose tissue are unclear.

### Summary

The present invention is defined by the appended claims. The present disclosure provides a therapeutic composition for controlling heat generation within an organism, including a molecule for adjusting binding of EBI-3 and p28 or binding of dimers of EBI-3 and p28 and receptors thereof, or a downstream signal pathway. In particular, it provides a molecule for use in reducing or increasing a body temperature of an organism.

The adjusting refers to inhibition or reduction of the binding of EBI-3 and p28, or inhibition or reduction of the binding of the dimers of EBI-3 and p28 and the receptors thereof, or down regulation of the downstream signal pathway, and the controlling refers to inhibition of heat generation, thereby reducing a body temperature of the organism; or the adjusting refers to promotion or enhancement of the binding of EBI-3 and p28, or promotion or enhancement of the binding of the dimers of EBI-3 and p28 and the receptors thereof, or up regulation of the downstream signal pathway, and the controlling refers to promotion of heat generation, thereby improving the body temperature of the organism.

Optionally, the molecule for use in reducing the body temperature of the organism includes a biological macromolecule or small molecule compound, comprising an anti-EBI-3 antibody, an anti-p28 antibody, an anti-IL-27R antibody, an EBI-3 soluble fragment, a p28 soluble fragment and an IL-27R soluble fragment.

Optionally, the molecule for use in increasing the body temperature of the organism is a recombinant IL-27 protein. In some embodiments, the molecule may further include a modifier of a recombinant IL-27 protein, and polypeptide or a small molecule compound which can activate activity of IL-27R.

The present disclosure further provides a method for screening a candidate compound capable of controlling heat generation within (i.e. reducing or increasing a body temperature of) an organism, including a step of measuring a binding condition of EBI-3 and p28, or a binding condition of dimers of EBI-3 and p28 and receptors thereof before and after application of a to-be-tested compound.

When the binding of EBI-3 and p28 is inhibited or reduced or the binding of the dimers of EBI-3 and p28 and the receptors thereof is inhibited or reduced after the to-be-tested compound is applied, the to-be-tested compound is identified as a candidate compound for inhibiting heat generation within the organism.

When the binding of EBI-3 and p28 is promoted or enhanced or the binding of the dimers of EBI-3 and p28 and the receptors thereof is promoted or enhanced after the to-be-tested compound is applied, the to-be-tested compound is identified as a candidate compound for promoting heat generation within the organism.

Optionally, the method includes the following steps:
(1) measuring a parameter of the binding of EBI-3 and p28 or a parameter of the binding of the dimers of EBI-3 and p28 and the receptors thereof in an organism model, so as to obtain a first measured value;
(2) after the to-be-tested compound is applied to the organism model, measuring the parameter of the binding of EBI-3 and p28 or the parameter of the binding of the dimers of EBI-3 and p28 and the receptors thereof, in the organism model, so as to obtain a second measured value; and
(3) comparing the first measured value and the second measured value.

Optionally, the method includes the following steps:
(1) measuring a parameter of the binding of EBI-3 and p28 or a parameter of the binding of the dimers of EBI-3 and p28 and the receptors thereof, in a first organism model, so as to obtain a first measured value;
(2) measuring a parameter of the binding of EBI-3 and p28 or a parameter of the binding of the dimers of EBI-3 and p28 and the receptors thereof, in a second organism model, so as to obtain a second measured value, wherein the first organism model and the second organism model belong to the same model, the to-be-tested compound is not applied to the first organism model, and the to-be-tested compound is applied to the second organism model; and
(3) comparing the first measured value and the second measured value.

Optionally, the method includes the following steps:
(1) measuring a parameter of the binding of EBI-3 and p28 or a parameter of the binding of the dimers of EBI-3 and p28 and the receptors thereof, in an organism model after the to-be-tested compound is applied, so as to obtain a measured value; and
(2) comparing the measured value with a reference value, wherein the reference value is a parameter of the binding of EBI-3 and p28 or a parameter of the binding of the dimers of EBI-3 and p28 and the receptors thereof, measured by the organism model when the to-be-tested compound is not applied.

Optionally, the organism model includes an animal model or a cell model.

The present disclosure further provides use of the composition in any one of the above descriptions in treatment of a metabolic disease.

Optionally, the metabolic disease includes hypothermia.

The present disclosure further provides a method for treating a metabolic disease, in which the composition in any one of the above descriptions is administered to the subject in need.

Optionally, the metabolic disease includes hypothermia.

### Brief Description of Drawings

FIG. 1 is a diagram of promotion of heat generation and energy expenditure by interleukin-27 (IL-27) signals.
FIG. 2 is a diagram in which IL-27 directly targets adipocytes to promote heat generation.
FIG. 3 is a diagram of promotion of heat generation by IL-27.
FIG. 4 shows reduction in energy expenditure and heat generation in IL-27 signal-deficient mice.
FIG. 5 shows that thermogenesis of IL-27Rα signals is not achieved by direct action on CD2+ lymphocytes or Lyz2+ myeloid cells.
FIG. 6 is a phenotype of an IL-27Rα KO and WT chimera in HFD-induced adaptive heat generation.
FIG. 7 shows that IL-27 up-regulates an uncoupling protein 1 (UCP1) and improves browning of subcutaneous white adipose tissue.
FIG. 8 shows that IL-27 promotes heat generation.

### Detailed Description

Various exemplary embodiments of the disclosure are described now, the detailed description should not be construed as limiting the disclosure but rather as a more detailed description of certain aspects, characteristics and implementations of the disclosure.

It is to be understood that the terminology used herein is for the purpose of describing particular embodiments only and is not intended to be limiting of the disclosure. In addition, for a numerical range in the disclosure, it is to be understood that the upper and lower limits of the range, and each intervening value therebetween, are specifically disclosed. Each smaller range between any stated value or any intervening value in a stated range and any other stated value or any other intervening value in the stated range is encompassed within the disclosure. The upper and lower limits of these smaller ranges may independently be included or excluded in the range.

Unless stated otherwise, all technical and scientific terms used herein have the same meaning as commonly understood by those skilled in the art to which the disclosure belongs. Although only preferred methods and materials are described herein, any methods and materials similar or equivalent to those described herein can be used in the implementation or testing of the disclosure. **In** case of conflict with any incorporated document, the specification will control. Unless state otherwise, "%" is a percent by weight.

As used herein, the term "heat generation" may be used interchangeably with the terms "heat production", "heat generation effect" and "thermogenesis". Thermogenesis used herein means a process in which mammals, including humans, regulate heat generation.

In the present disclosure, binding of an EB virus-induced gene 3 (EBI-3) and a subunit of cytokine IL-27 secreted by dendritic cells (p28) means that an EBI-3 subunit and a p28 subunit form a heterodimeric structure with a certain spatial arrangement by interaction which may be connection by a disulfide bond.

The term "receptor" as used herein refers to a biological macromolecule capable of being bound to the above-mentioned dimers and of causing a change in cell functions. The receptor contains at least an active site which recognizes and is bound to the dimer and a functional active site responsible for generating a response reaction, and thus initiates a series of biochemical reactions, ultimately leading to generation of a biological effect by a target cell. The binding between the receptor and the dimer in the disclosure results in activation of the receptor and creates a basic step of activating subsequent signaling by the receptor. Under physiological conditions, the binding between the receptor and the dimer is not mediated by covalent bonds, but rather is bounded to each other primarily by ionic bonds, hydrogen bonds, Van der Waals forces, and hydrophobic interaction.

The term "signal pathway" as used herein refers to a phenomenon that when a certain reaction occurs in a cell, a signal transmits information from an exterior of the cell to an interior of the cell, and the cell responds according to the information. The signal pathway in the disclosure particularly refers to a series of enzymatic reaction pathways which can transmit extracellular molecular signals into the cell through a cell membrane to exert an effect.

Some embodiments of the present disclosure provide a composition for controlling heat generation within an organism.

The present disclosure provides a composition for controlling heat generation within an organism, including a molecule for adjusting binding of EBI-3 and p28 or binding of dimers of EBI-3 and p28 and receptors thereof, or a downstream signal pathway.

In some embodiments, the adjusting refers to inhibition or reduction of the binding of EBI-3 and p28, or inhibition or reduction of the binding of the dimers of EBI-3 and p28 and the receptors thereof, or down regulation of the downstream signal pathway, and the controlling refers to inhibition of heat generation, thereby reducing a body temperature of the organism.

In some embodiments, the molecule includes a biological macromolecule or small molecule compound, comprising an anti-EBI-3 antibody, an anti-p28 antibody, an anti-IL-27R antibody, an EBI-3 soluble fragment, a p28 soluble fragment and an IL-27R soluble fragment.

In some embodiments, the adjusting refers to promotion or enhancement of the binding of EBI-3 and p28, or promotion or enhancement of the binding of the dimers of EBI-3 and p28 and the receptors thereof, or up regulation of the downstream signal pathway, and the controlling refers to promotion of heat generation, thereby improving the body temperature of the organism.

In some embodiments, the molecule includes a recombinant IL-27 protein, a modifier thereof, and polypeptide or a small molecule compound which can activate activity of IL-27R.

Optionally, the composition according to the disclosure includes a molecule for adjusting IL27 signal or binding of IL27 to a receptor thereof or the downstream signal pathway. The downstream signal pathway includes, but is not limited to, JAK/STAT3, p38 MAPK, or the like. In the present disclosure, thermogenesis and energy expenditure are mediated by adipocytes which include, but are not limited to, brown/beige adipocytes and white adipocytes. In the present disclosure, the above-mentioned adipocytes act as IL27 target cells, not immune cells.

Some embodiments of the present disclosure provide a method for screening a candidate compound capable of controlling heat generation within an organism.

In the present disclosure, the screening method at least includes a step of measuring, using a reagent, a binding condition of EBI-3 and p28, or a binding condition of dimers of EBI-3 and p28 and receptors thereof, or a downstream signal pathway triggered by the binding before and after application of the to-be-tested compound. The above binding condition is further measured by a step of measuring a relevant parameter using a reagent, wherein the parameter includes a quantity of the dimer formed by EBI-3 and p28, or a metabolic phenotype, or a quantity of a thermogenic protein. The thermogenic protein includes, but is not limited to, UCP1, a peroxisome proliferator-activated receptor (PPARα) and a peroxisome proliferator-activated receptor-γ co-activator-1α (PGC-1α). In the present disclosure, activation (also called as excitation sometimes) of the downstream signal pathway refers to further expression of a downstream target gene. Optionally, the reagent includes a sequence shown by SEQ ID NO: 1-30.

In some embodiments, when the binding of EBI-3 and p28 is inhibited or reduced or the binding of the dimers of EBI-3 and p28 and the receptors thereof is inhibited or reduced or a downstream signal triggered by the binding is downregulated after the to-be-tested compound is applied, the to-be-tested compound is identified as a candidate compound for inhibiting heat generation within the organism.

In some embodiments, when the binding of EBI-3 and p28 is promoted or enhanced or the binding of the dimers of EBI-3 and p28 and the receptors thereof is promoted or enhanced or the downstream signal triggered by the binding is up-regulated after the to-be-tested compound is applied, the to-be-tested compound is identified as a candidate compound for promoting heat generation within the organism.

In some embodiments, the method includes the following steps:
(1) measuring a parameter of the binding of EBI-3 and p28 or a parameter of the binding of the dimers of EBI-3 and p28 and the receptors thereof, or a parameter of an activation condition of the downstream signal pathway in an organism model, so as to obtain a first measured value;
(2) after the to-be-tested compound is applied to the organism model, measuring the parameter of the binding of EBI-3 and p28 or the parameter of the binding of the dimers of EBI-3 and p28 and the receptors thereof, or the parameter of the activation condition of the downstream signal pathway in the organism model, so as to obtain a second measured value; and
(3) comparing the first measured value and the second measured value.

In some embodiments, the method includes the following steps:
(1) measuring a parameter of the binding of EBI-3 and p28 or a parameter of the binding of the dimers of EBI-3 and p28 and the receptors thereof, or a parameter of an activation condition of the downstream signal pathway in a first organism model, so as to obtain a first measured value;
(2) measuring a parameter of the binding of EBI-3 and p28 or a parameter of the binding of the dimers of EBI-3 and p28 and the receptors thereof, or a parameter of an activation condition of the downstream signal pathway in a second organism model, so as to obtain a second measured value, wherein the first organism model and the second organism model belong to the same model, the to-be-tested compound is not applied to the first organism model, and the to-be-tested compound is applied to the second organism model; and
(3) comparing the first measured value and the second measured value.

In some embodiments, the method includes the following steps:
(1) measuring a parameter of the binding of EBI-3 and p28 or a parameter of the binding of the dimers of EBI-3 and p28 and the receptors thereof, or a parameter of an activation condition of the downstream signal pathway in an organism model after the to-be-tested compound is applied, so as to obtain a measured value; and
(2) comparing the measured value with a reference value, wherein the reference value is a parameter of the binding of EBI-3 and p28 or a parameter of the binding of the dimers of EBI-3 and p28 and the receptors thereof, or a parameter of an activation condition of the downstream signal pathway measured by the organism model when the to-be-tested compound is not applied.

In some embodiments, the organism model includes an animal model or a cell model.

The present disclosure further provides use of the composition in any one of the above descriptions in treatment of a metabolic disease.

The present disclosure further provides a method for treating a metabolic disease, in which the composition in any one of the above descriptions is administered to the subject in need.

In some embodiments, the metabolic disease includes hypothermia.

The present disclosure discloses functions of the IL-27(EBI-3/p28) signals in the improvement of in vivo heat generation. It is proved that IL-27 directly acts on the adipocytes, activates the p38 MAPK-PGC1 signals and stimulates generation of UCP1. Therapeutic administration of IL-27 may reverse hypothermia of EBI-3 KO mice induced by the low temperature.

The present disclosure provides a composition and a method for controlling heat generation within an organism, and a use. Research of the present disclosure finds that (EBI-3/p28) signals have an important function in an improvement of in vivo heat generation, and proves that IL-27 directly acts on adipocytes, activates p38 MAPK-PGC1 signals and stimulates generation of UCP1.

### Example

### 1. Experimental material

### 1.1 Material

### 1.1.1 Mouse

Interleukin-27Rα KO (B6N.129P2-*II27ra^{tm1Mak}*/J, No.: 018078) and EBI-3 KO (B6.129X1-Ebi3*^{tm1Rsb}*/J, No.: 008691) mice were purchased from Jackson Laboratory. Adipoq-cre mice were supplied by Professor Yong Liu. IL-27Rα^{f/f} mice [herein] were prepared by our own laboratory. Animal experiments were conducted in accordance with ethical specifications and protocols approved by the institutional animal protection and utilization committee of Jinan University and the institutional animal care and utilization committee of Yale University. Groups of mice with matched genders and ages were used in all experiments, with 4 to 6 animals per cage. IL-27 treatment experiments and bone marrow chimera production were performed in random groups. The groups of mice were bred in a specific sterile animal facility with a controlled temperature and humidity, the temperature was 25°C, a cycle of light and dark was 12h:12h, and food and water were freely obtained.

### 1.1.2 Reagent

Human serum samples were analyzed on a Bio-Plex system (BioRad) using a Bio-Plex-Pro^{™} Human Cytokine 17-Plex analysis kit (Cat. No.M5000031YV) and a Bio-Plex Pro^{™} Human Inflammation Panel 1 (Cat. No.171-AL001M). A human IL-27 ELISA kit (434607) was purchased from Biolegend. Glucose (63005518) was purchased from Chinese Sinopharm Chemical Reagent Company. Blood glucose test strips (one-touch) were purchased from Johnson and Johnson. An anti-mouse pAKT monoclonal antibody (4060), an anti-mouse AKT monoclonal antibody (4685), an anti-mouse GAPDH monoclonal antibody (5174), an anti-mouse HSP90 monoclonal antibody (4877), an anti-mouse PPARγ monoclonal antibody (2435), an anti-mouse pSTAT3 monoclonal antibody (9145), an anti-mouse STAT3 monoclonal antibody (9139), an anti-mouse p-p38 MAPK (9211), an anti-mouse p38 MAPK (9212), an anti-mouse pATF2 monoclonal antibody (9221), and an anti-mouse ATF2 monoclonal antibody (35031) were purchased from Cell Signaling Technology. An anti-mouse UCP1 monoclonal antibody (ab10983), an anti-mouse PGC-1α monoclonal antibody (ab54481), an anti-mouse PPARα monoclonal antibody (ab8934), an anti-mouse PRDM16 monoclonal antibody (ab202344) and an anti-mouse IL-27Rα monoclonal antibody (ab5997) were purchased from Abcam. APC-CY7-anti-mouse CD45 (clone 30-F11) was purchased from BD. APC anti-mouse CD11b (clone M1/70) and PE anti-mouse F4/80 (clone BM8) were purchased from Sungene Biotech (Tianjin, China). Recombinant mice IL-27 (rmIL-27) (577408) were purchased from Biolegend. An STAT3 inhibitor SH-4-54 and a p38 MAPK inhibitor SB203580 were purchased from Selleck Chemicals. Brown/beige fat differentiation agent dexamethasone (D4902), rosiglitazone (R2408), indomethacin (I7378), isoproterenol (15627), forskolin (F6886), 3-isobutyl-1-methylxanthine (IBMX) (15879), a triglyceride detection kit (T2449) and insulin (13536) were purchased from Sigma. A leptin detection kit (MOB00) and an adiponectin detection kit (MRP300) were purchased from R and D. An insulin detection kit (90080) was purchased from Crystal Chem.

### 2. Experimental method

### 2.1 Enzyme-linked immunosorbent assay

Human serum was obtained as described above. Mouse serum was collected after high fat diet (HFD) feeding for 10 weeks. Human IL-27 and mouse insulin were detected using the ELISA kit according to an operation instruction.

### 2.2 Luminex immunoassay

Human serum was collected as described above. Detection of human serum inflammatory factors was performed on the Bio-Plex 200 flow liquid chip analysis system (BioRad) using the Bio-Plex-Pro^{™} Human Cytokine 17-Plex analysis kit (Cat. No.M5000031YV) and the Bio-Plex Pro^{™} Human Inflammation Panel 1 (Cat. No.171-AL001M) according to instructions of the user manual.

### 2.3 Western blot

A whole cell lysate or tissue lysate was extracted using an RIPA lysis buffer (Beyotime, China) supplemented with a complete protease inhibitor (Roche) and a phosphatase inhibitor Cocktail 2 (P5726, Sigma), and a supernatant was used for subsequent analysis. Proteins were diluted in a Loading dye (BL502A, Bio-sharp), heated at 95°C for 10min, and separated on 4-12% polyacrylamide gel. The proteins were transferred to polyvinylidene fluoride membranes and western blot experiments were performed with the commercial antibodies and methods-reagents mentioned in the drawing.

### 2.4 Histology

Adipose or liver tissue was fixed in 4% paraformaldehyde/1XPBS overnight at 4°C and embedded in paraffin before sectioning. Sections were stained with hematoxylin and eosin (H and E) or oil red O (liver) and photographed under a bright field microscope.

### 2.5 Immunohistochemistry

UCP1 and immunohistochemistry were performed using a rabbit specific HRP/DAB (ABC) detection IHC kit (ab64261, Abcam) according to instructions of the manufacturer.

### 2.6 Core body temperature measurement and cold stimulation experiment

Cold stimulation experiments were performed in a temperature controlled cold room. Mice were placed individually in individual cages (without bedding) at 4°C. The mice had free access to food and water. Rectal core body temperatures were recorded every 2 hours using a digital thermometer and a rectal thermocouple probe (TH-212, HICHANCE, China). If the core body temperature fell below 20°C, the individual mice were euthanized and scored as events for survival analysis.

### 2.7 Metabolic cage

Energy loss and energy expenditure of the mice were measured using a comprehensive laboratory animal monitoring system (CLAMS, Columbus Instruments, Columbus, OH) metabolic cage in the environmental control rodent incubator of Yale University. The mice were bred individually and acclimated in metabolic chambers for 48 hours prior to data collection. The mice had free access to food and water. Each mouse was subjected to continuous monitoring of physical activity and food intake. During the experiment, concentrations of CO₂/O₂ were collected 4 times per hour for each mouse.

### 2.8 RNA extraction and gene expression analysis

Total RNA was extracted from frozen tissue using a TRNzol general reagent (Tiangen, Beijing) and quantified using a Nanodrop 2000 ultraviolet-visible spectrophotometer (Thermo). The cDNA was prepared by 1µg of total RNA through a reverse transcription polymerase chain reaction using a PrimeScript^{™} RT Reagent kit (TAKARA, Dalian). Real-time quantitative PCR of the cDNA was performed on a CFX96^{™} real-time PCR detection system (Bio-Rad) using a TB Green^{™} Premix-EX-Taq^{™} II kit (TAKARA). A fold change in expression was calculated by a △△Cₜ method using immunohistochemistry with mouse HPRT as an internal reference. Primer pair sequences were as follows.
Mouse HPRT: forward-TCATTATGCCGAGGATTTG (SEQ ID NO. 1) and reverse-GCCTCCCATCTCCTTCAT (SEQ ID NO. 2);
TNF-α: forward-CTACTGAACTTCGGGGTGAT (SEQ ID NO. 3) and reverse-CAGGCTTGTCACTCGAATT (SEQ ID NO. 4);
IL-6: forward-TCCAGTTGCCTTCTTGGGAC (SEQ ID NO. 5), and reverse-GTGTAATTAAGCGCCGACTTG (SEQ ID NO. 6);
IL-12p40: forward-GGCTGGTGCAAAGAAACATGGACTTGA (SEQ ID NO. 7), and reverse-TGCAGACAGAGACGCCATTCCACAT (SEQ ID NO. 8);
IL-1β: forward-CAACCAACAAGTGATATTCTCCATG (SEQ ID NO. 9), and reverse-GATCCACACTCTCCAGCTGCA (SEQ ID NO. 10);
IL-4: forward-GAAAACTCCATGCTTGAAGAA (SEQ ID NO. 11), and reverse-TCTTTCAGTGATGTGGACTTG (SEQ ID NO. 12);
IL-5: forward-TCACCGAGCTCTGTTGACAA (SEQ ID NO. 13), and reverse-CCACACTTCTCTTTTTGGCG (SEQ ID NO. 14);
IL-13: forward-TGAGCAACATCACACAAGACC (SEQ ID NO. 15), and reverse-GGCCTTGCGGTTACAGAGG (SEQ ID NO. 16);
Ucp1: forward-ACTGCCACACCTCCAGTCATT (SEQ ID NO. 17), and reverse-CTTTGCCTCACTCAGGATTGG (SEQ ID NO. 18);
Cox8b: forward-GAACCATGAAGCCAACGACT (SEQ ID NO. 19), and reverse-GCGAAGTTCACAGTGGTTCC (SEQ ID NO. 20);
Cidea: forward-TGCTCTTCTGTATCGCCCAGT (SEQ ID NO. 21), and reverse-GCCGTGTTAAGGAATCTGCTG (SEQ ID NO. 22);
Prdm16: forward-CAGCACGGTGAAGCCATTC (SEQ ID NO. 23), and reverse-GCGTGCATCCGCTTGTG (SEQ ID NO. 24);
Adiponectin: forward-GAATCATTATGACGGCAGCA (SEQ ID NO. 25), and reverse-TCATGTACACCGTGATGTGGTA (SEQ ID NO. 26); and
Elovl3: forward-TCCGCGTTCTCATGTAGGTCT (SEQ ID NO. 27), and reverse-GGACCTGATGCAACCCTATGA (SEQ ID NO. 28).

### 2.9 Bone marrow chimera

IL-27Rα KO and WT mice which were 10 weeks old were used as bone marrow cell donors. Bone marrow was separated from hind limb femurs, erythrocytes were lysed, a product was filtered by a cell filter (40µm), and cell pellets were washed 3 times with sterile PBS, counted and stored on ice for injection. The IL-27Rα KO and WT mice which were 10 weeks old were lethally irradiated (900rads), 1X10⁷ bone marrow cells were transplanted by ocular intravenous injection, the mice were placed in specific pathogen-free facilities and supplemented with sterile water and feed for 8 weeks to reconstitute immune systems, and then, metabolic research was performed.

### 2.10 Primary beige adipocyte preparation

Inguinal subcutaneous adipose tissue was minced and digested at 37°C for 45min in PBS containing collagenase II (1mg/ml), and a tissue suspension was filtered by a 100µm cell filter and centrifuged at 600g for 5min, such that a stromal vascular fraction (SVF) was granulated. After granulation, the product was further filtered by a 40µm cell filter and placed on a collagen-coated plate. After overnight culturing, a supernatant containing non-adherent cells was removed. Precursor adipocytes were grown in DMEM containing 10% FBS and insulin (5µg/ml) for fusion. The product was treated for 2 days with dexamethasone (1µM), 3-isobutyl-1-methylxanthine (IBMX, 0.5mM), insulin (5µg/ml), indomethacin (125nM) and rosiglitazone (1µM) to induce differentiation of the fused cells, and then independently treated for another 5 days with insulin (5µg/ml) and triiodothyronine (T3, 1nM). On day 7, the cells were pretreated overnight with and without IL-27 (100ng/ml) and then treated with isoproterenol (10µM) or forskolin (10µM) for 4-6 hours. IL-27 (100ng/ml) was added for treatment for 0-120min on day 7 for protein phosphorylation analysis, or for 12-24h for protein expression level detection. For signal inhibition experiments, either the STAT3 inhibitor (C188-9, 10µM) or the p38 MAPK inhibitor (SB203580, 10µM) was added to a medium 0.5h prior to and during IL-27 treatment.

### 2.11 Flow cytometric analysis (FACS)

Epididymal adipose tissue was minced and digested as described above. SVF particles were used for the surface staining of the APC-CY7 anti-mouse CD45, APC anti-mouse CD11b, PE anti-mouse F4/80, the product was incubated for 15min, and then the cells were washed with PBS and analyzed using a BD-FACSVerse flow cytometer (BD).

2.12 Production of IL-27Rα conditional knockout mouse. A development of IL-27Rα conditional knockout mice was performed using a gene targeting technology. Briefly, a 997bps CKO region containing exon 3 and exon 4 of the *IL27ra* gene (GenBank NM_016671.3), a 5.2kb 5' homology arm and a 3kb 3' homology arm were amplified from BAC clones using a high fidelity Taq technology, and the resultant, a recombination site and a selectable marker were assembled into a targeting vector. The final targeting vector was determined by multiple times of digestion by restriction endonuclease and full sequence analysis and transformed into 129s mouse embryonic stem cells by electroporation. A correct embryonic stem cell clone was identified and injected into a C57BL/6J mouse blastocyst. After a chimera mouse was unambiguously identified, the chimera mouse and a C57BL/6J mouse were backcrossed. Genotyping PCR confirmed germ line transmission. Then, mice with an IL-27Rα f/w genotype and B6 adipoq-cre mice were bred. Mice with a desired genotype were selected for the experiment. Genotyping primers at flox or wt were detected: forward: CTGGTTCTGGTATGGTTTGGGGTT (SEQ ID NO. 29) and reverse: TGAAAGAACTCAACAGTGGGCCGG (SEQ ID NO. 30).

### 2.13 IL-27 treatment

8-week-old WT mice were fed with HFD for 32 weeks and randomly divided into 2 groups. The mice were injected intraperitoneally with rmIL-27 (100µg/kg) or PBS for 15 consecutive days, and then, metabolic analysis was performed. 12-month-old EBI-3 KO mice were randomly divided into 2 groups, and injected intraperitoneally with rmIL-27 (100µg/kg) or PBS for 7 consecutive days. The mice were then subjected to a cold stimulation experiment at 4°C.

### 2.14 Statistical analysis

Mapping and statistical analysis were performed using Graphpad Prism software (version 7). All statistical tests were fully described in the graph legend and met the criteria for normal distribution with similar variances. A sample size was not predetermined using a statistical method. Comparison between the two groups was performed using a t-test. For evaluation data of relevant samples, repeated measurement analysis of variance (AVOVA) was performed. For an evaluation of more than two groups, one-way analysis of variance and multiple comparison were used. For an evaluation between two independent variables, a two-way analysis of variance with multiple comparison was used. Survival analysis was tested using a log rank. Linear regression analysis was adopted for correlation analysis. The data was expressed by a mean ± s.e.m., unless stated otherwise. P<0.05 was considered to be statistically significant, and denoted as *p<0.05, **p<0.01, **p<0.001, and NS meant "not significant".

### 3. Experiment result

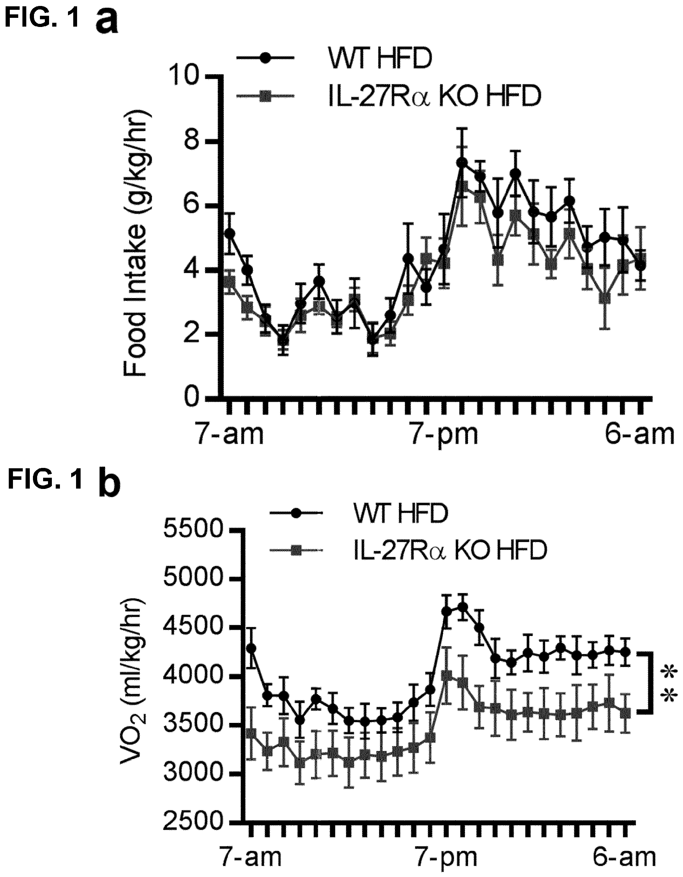

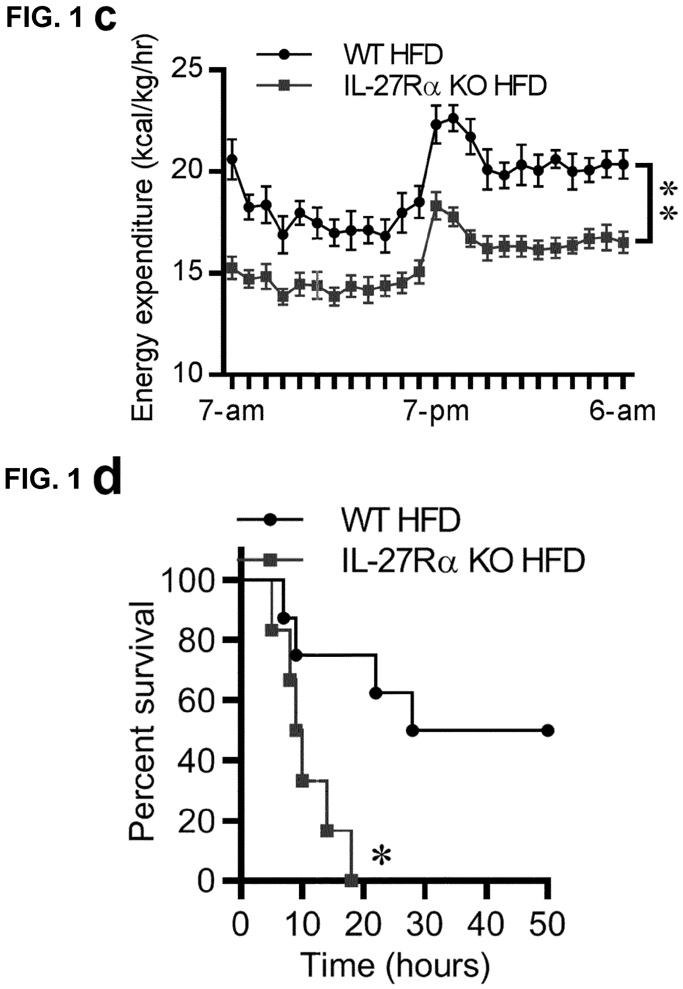

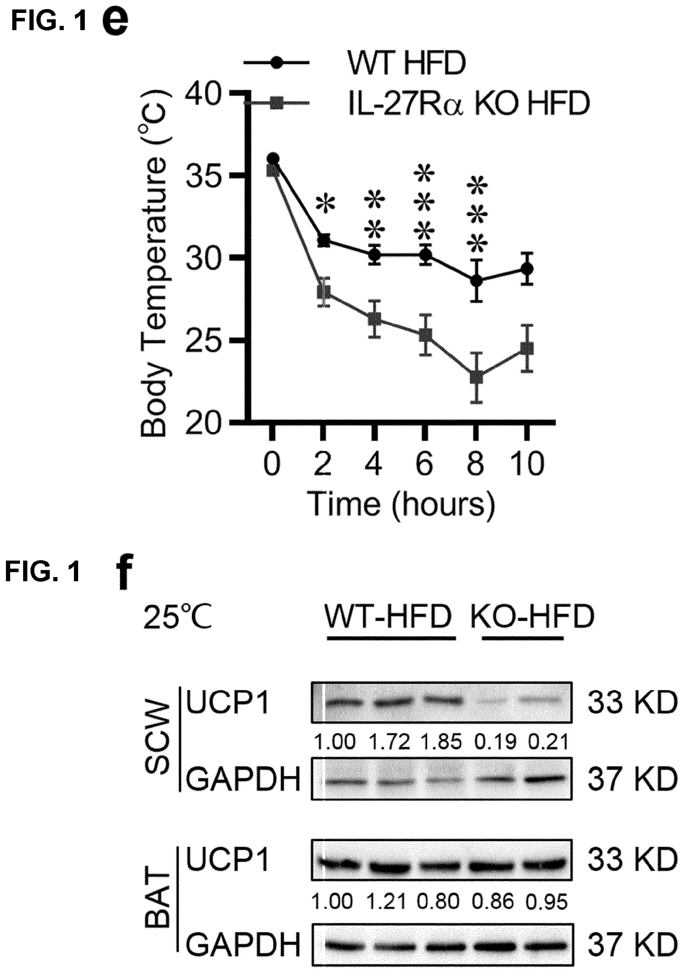

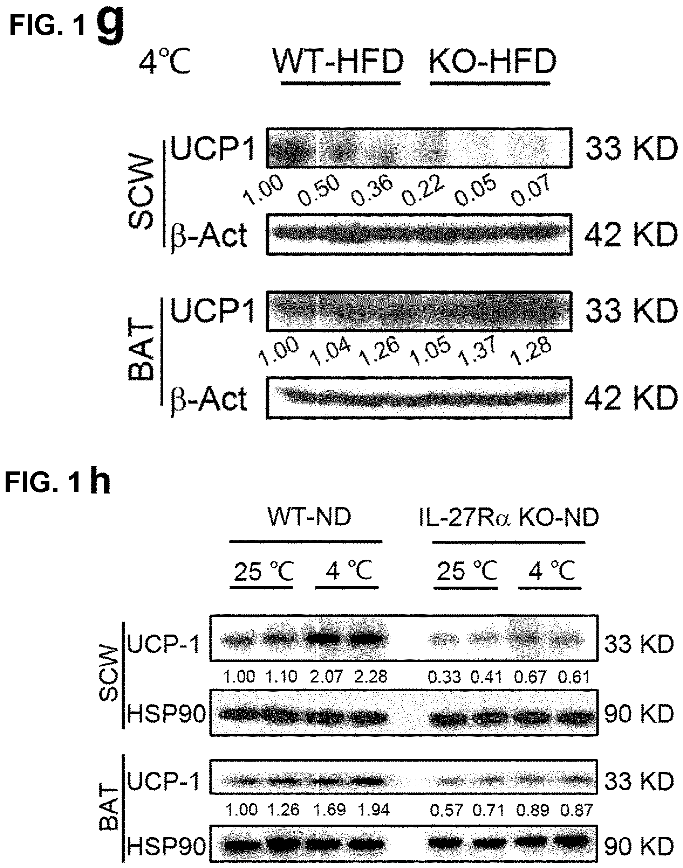

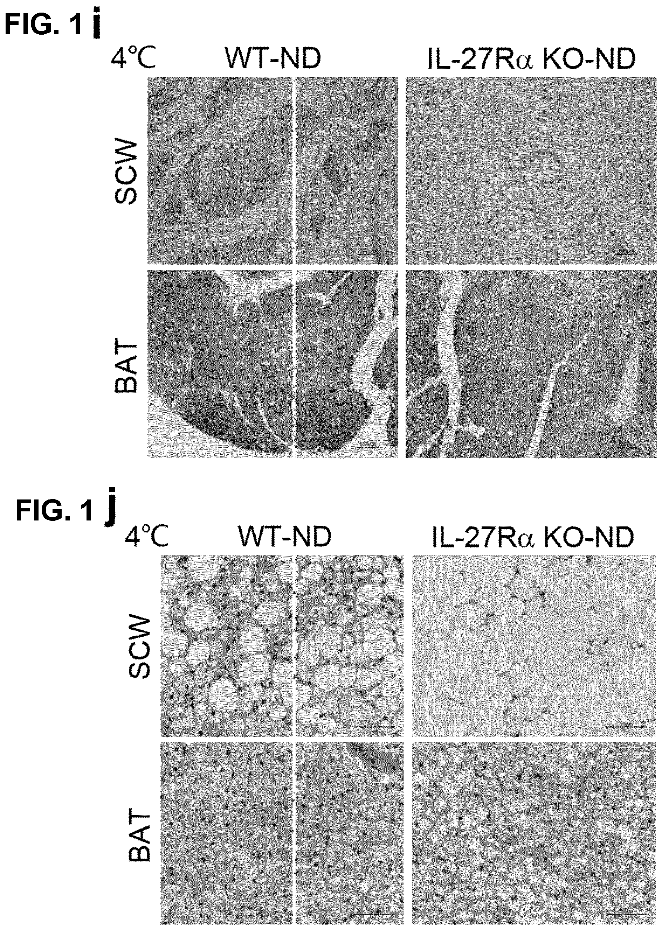

FIG. 1 is a diagram of promotion of heat generation and energy expenditure by IL-27 signals. As for a-c: IL-27Rα KO mice and WT control mice were fed with HFD for 4 weeks and then bred in metabolic cages. Food intake (shown in a in the drawing), oxygen consumption (shown in b in the drawing) and energy expenditure (shown in c in the drawing) were monitored in 24 hours (n=7-8). As for d and e: IL-27Rα KO mice and WT control mice were fed with HFD for 6 weeks and then subjected to cold stimulation (4°C). Survival curves were shown in the drawing (d, n=6-8), and rectal temperatures were shown in the drawing (e, n=11-15). As for f and g: IL-27Rα KO and WT mice were fed with HFD for 6 weeks, and then stayed at 25°C (shown in f in the drawing) or were subjected to cold stimulation at 4°C (shown in g in the drawing) for 2 hours. Subcutaneous fat (SCW) and brown adipose tissue (BAT) were collected for UCP1 immunoblot analysis. As for h-j: IL-27Rα KO and WT mice fed with a normal diet stayed at 25°C or were subjected to cold stimulation at 4°C for 48 hours. SCW and BAT were collected for UCP1 immunoblot analysis (shown in h in the drawing), i was a UCP1 immunohistochemical staining result (scale=100µm), and j was H and E staining of BAT and SCW tissue (scale=50µm). As for (f-h): a band density was quantified and normalized with ImageJ. Each lane represented a biologically independent sample, and the experiment was repeated at least twice with similar results. Data was a mean of the biologically independent samples ± s.e.m., and two-way analysis of variance (a-c and e) was shown; and a log rank test (d) was shown. **p*<0.05, ***p*<0.01, and ****p*<0.001.

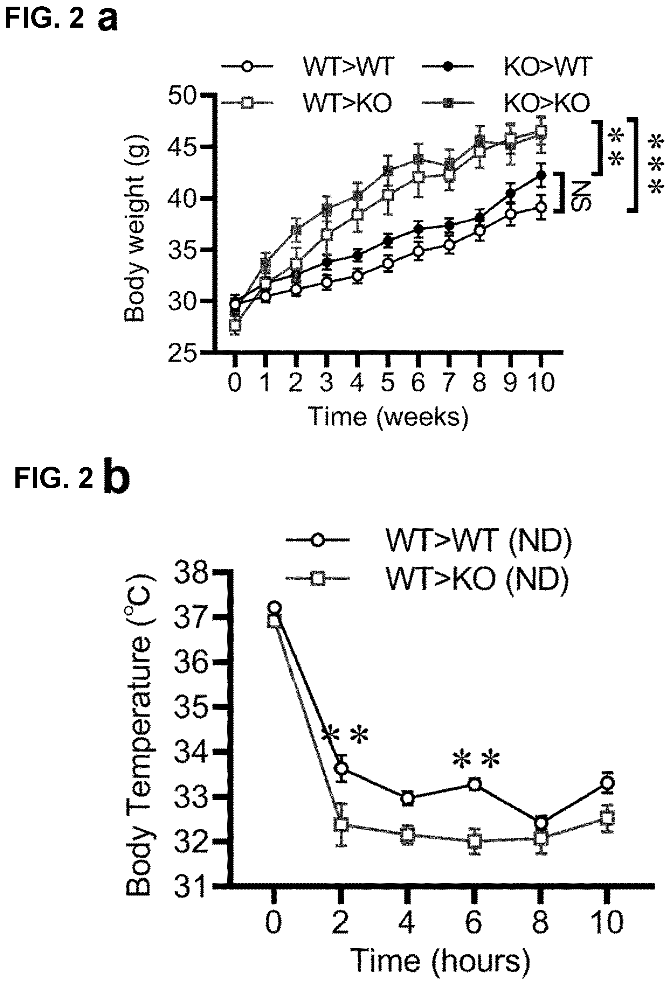

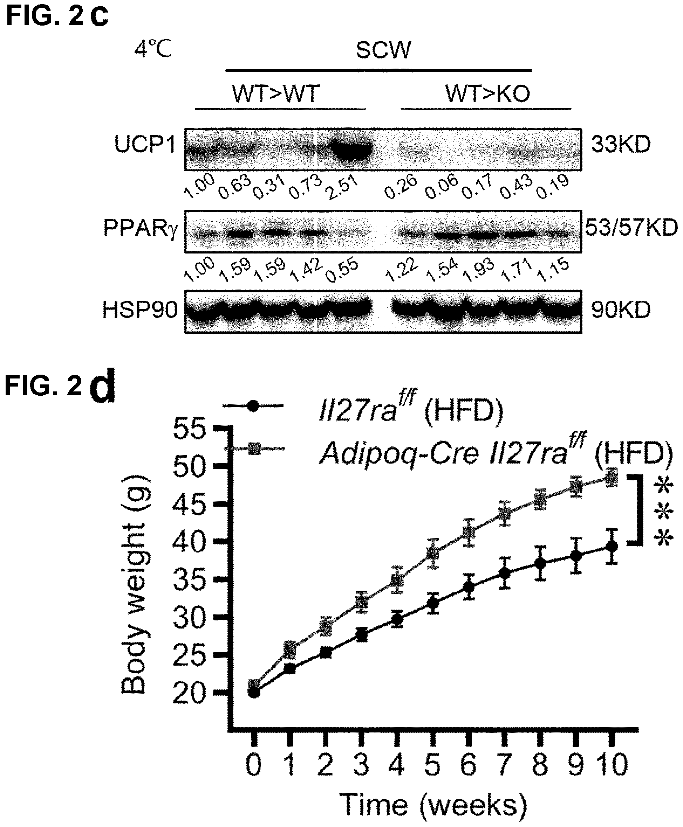

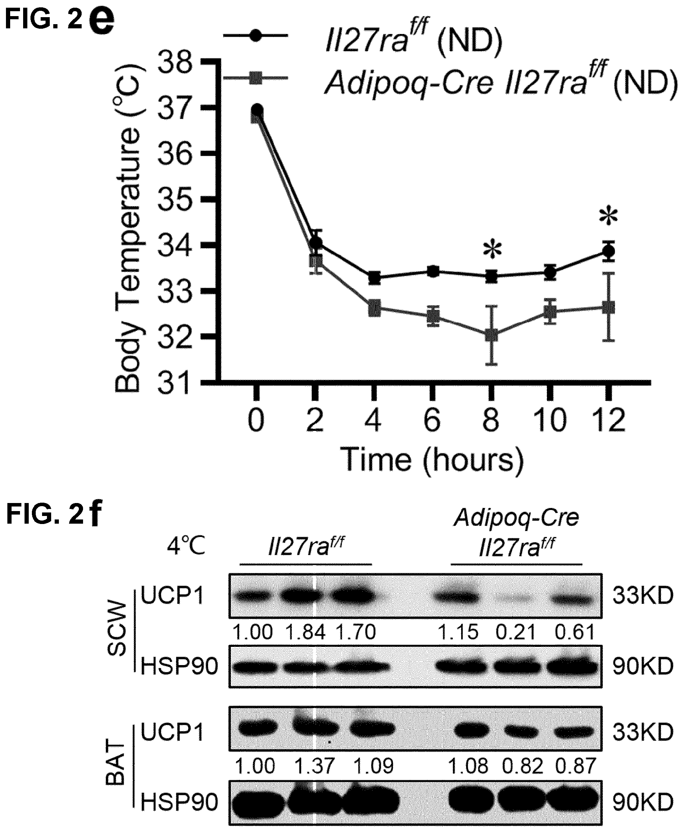

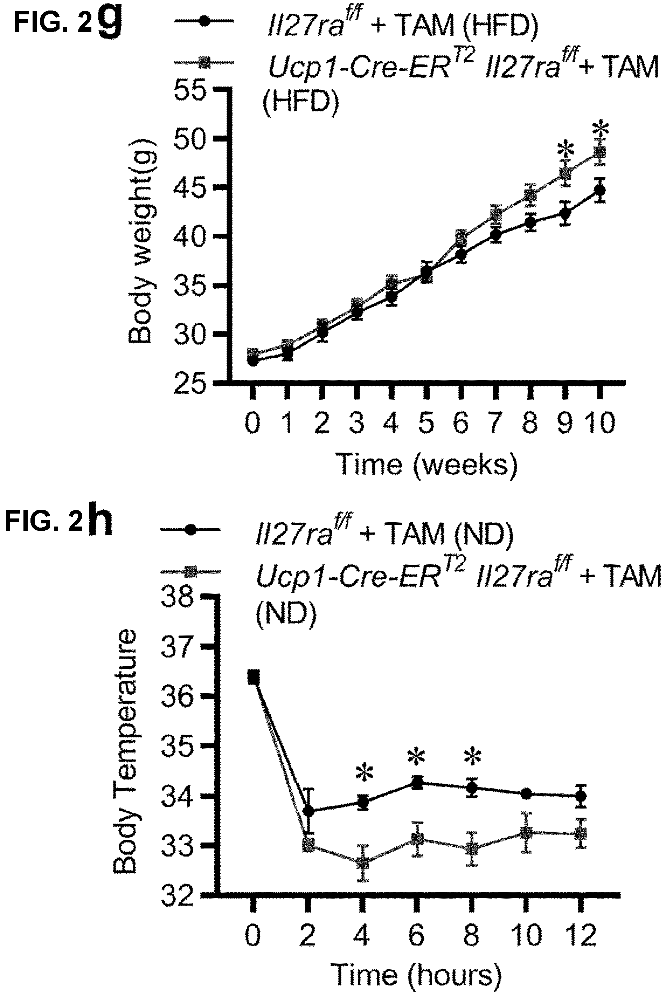

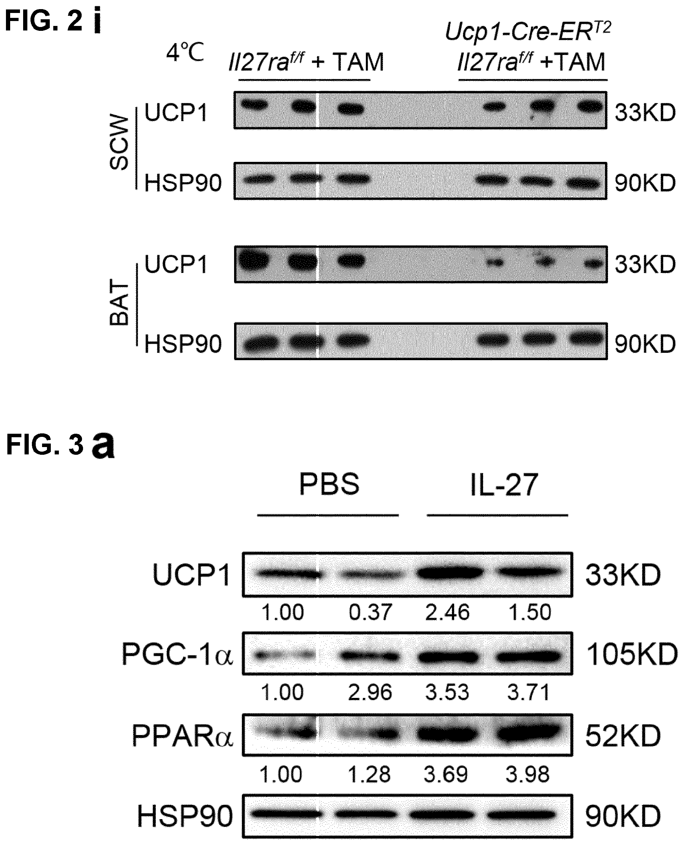

FIG. 2 is a diagram in which IL-27 directly targets adipocytes to promote heat generation. As for a: IL-27Rα KO or WT mice were fed with HFD for 10 weeks as donors or receptors to generate four groups of bone marrow chimeras. Body weight was recorded weekly (n=10-13). As for b: an influence of cold stimulation on rectal temperatures of chimera mice fed with a normal diet (4°C, n=6-7) was shown. As for c: after the chimera mice fed with the normal diet were subjected to cold stimulation at 4°C for 12 hours, SCW was collected for an immunoblot. As for d. 8-week-old *Adipoq-Cre II27ra*^{f/f} mice and an *II27ra*^{f/f} control group were fed with HFD for 10 weeks. Body weight was recorded weekly (n=7-9). As for e: an influence of cold stimulation on rectal temperatures of mice fed with a normal diet (4°C, n=16-17) was shown. As for f: after the *Adipoq-Cre II27ra*^{f/f} and *II27ra*^{f/f} mice fed with the normal diet were subjected to cold stimulation at 4°C for 12 hours, SCW and BAT were collected for an immunoblot. As for g. 8-week-old *Ucp1-Cre II27ra*^{f/f} mice and an *II27ra*^{f/f} control group were fed with HFD for 10 weeks. Body weight was recorded weekly (n=5-6). As for h: an influence of cold stimulation on rectal temperatures of mice fed with a normal diet (4°C, n=5-8) was shown. As for i: after the *Ucp1-Cre II27ra*^{f/f} and *II27ra*^{f/f} mice fed with the normal diet were subjected to cold stimulation at 4°C for 12 hours, SCW and BAT were collected for an immunoblot (c, f and i), and a band density was quantified and normalized with ImageJ. Each lane represented an independent biological sample, and the experiment was repeated at least twice with similar results. Data was a mean of the biologically independent samples ± s.e.m. Two-way analysis of variance (a, b, d, e, g and h) was shown. *p<0.05, ***p*<0.01, and ***p*<0.001.

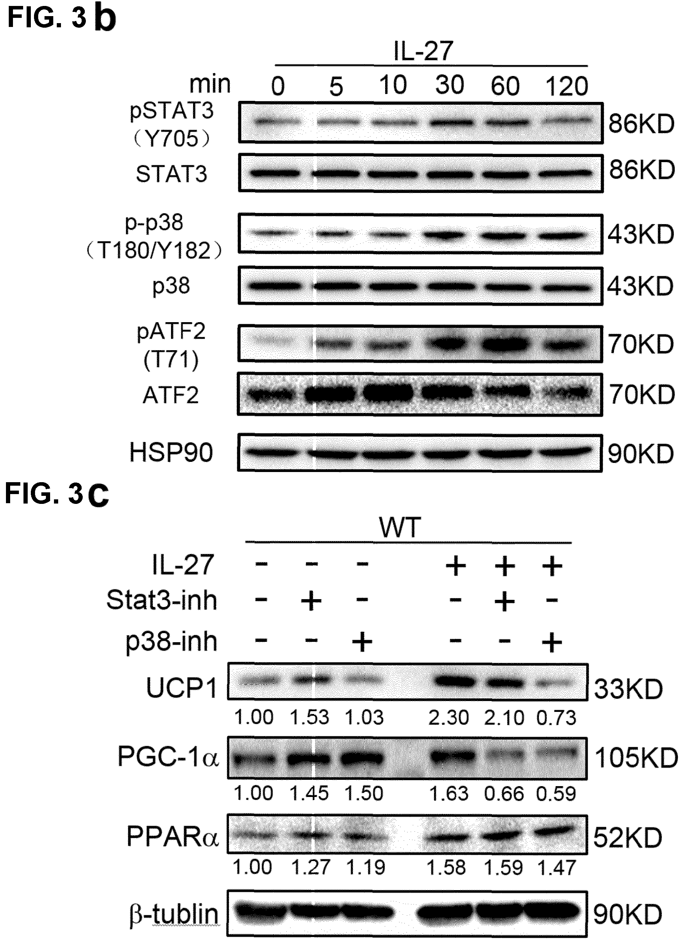

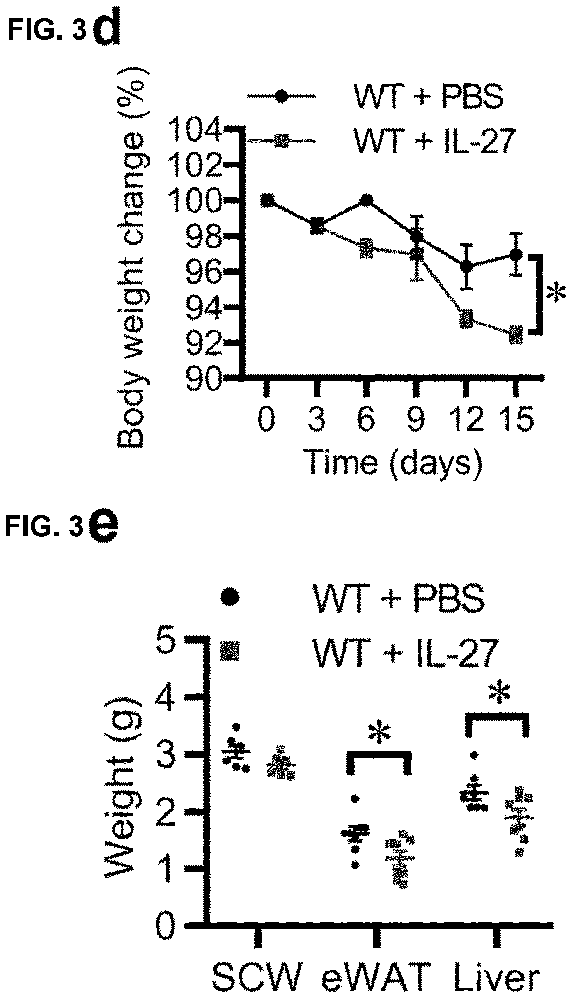

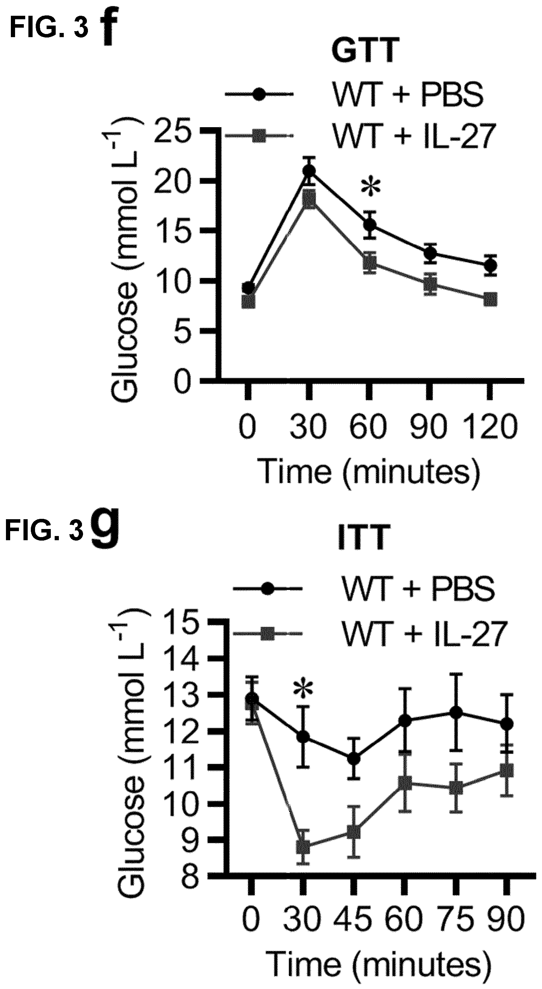

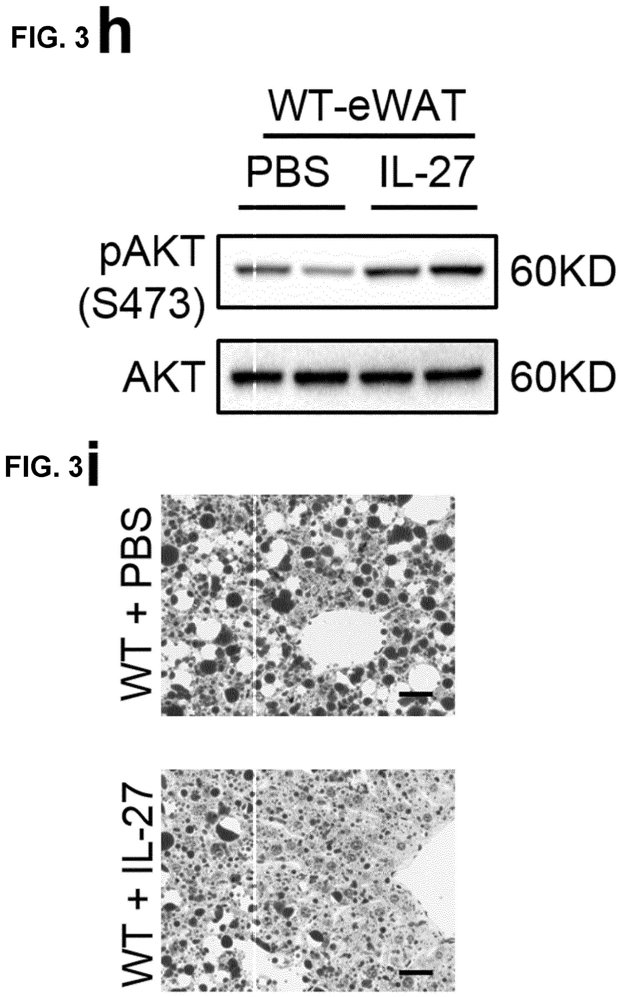

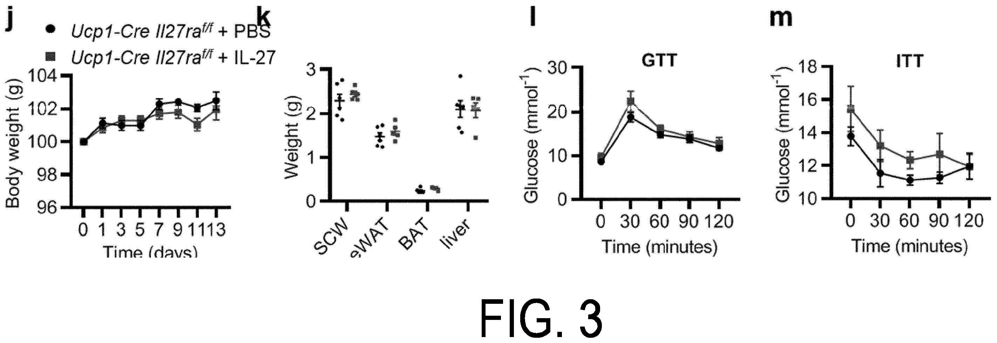

FIG. 3 shows promotion of heat generation by IL-27. As for a: primary beige adipocytes were generated in vitro from an SVF of WT SCW and then treated with rmIL-27 (100ng/ml) or PBS for 24 hours. Cells were lysed and a protein extract was used for immunoblot analysis. As for b: immunoblot analysis of phosphorylation of the protein in the WT primary beige adipocyte extract treated with the rmIL-27 (100ng/ml) for a specified time was shown. As for c: the primary beige adipocytes derived from WT SCW were generated in vitro. The STAT3 inhibitor (C188-9, 10µM) or p38 MAPK inhibitor (SB203580, 10µM) was added to a medium 0.5 hours prior to culturing and in duration of rmIL-27 treatment (100ng/ml for 12 hours). Cell lysis and the representative immunoblot of the protein extract were shown. As for d-j: WT mice were fed with HFD for 32 weeks and then injected intraperitoneally. Every other day, rmIL-27 (100µg/kg) or PBS was injected for 15 days. As for d: body weight was recorded at specified time points (n=8). As for e: after rmIL-27 treatment for 15 days, adipose tissue was collected and weighed (n=6-8) for a glucose tolerance test (f, n=8) and an insulin tolerance test (g, n=8). As for h: the mice were fasted overnight and then injected intraperitoneally with 0.75U/kg insulin (15min). Immunoblot analysis of pAKT (pSer473) in epididymal fat was performed. As for i: after rmIL-27 treatment for 15 days, oil-red O liver tissue staining was performed, with the scale=50µm. As for j-m: UCP1-KO mice were fed with HFD for 20 weeks and then injected intraperitoneally with rmIL-27 (100µg/kg) or PBS every other day for 15 days. As for j: body weight was recorded at specified time points (n=6). As for k: adipose tissue and livers were collected and weighed after rmIL-27 treatment for 15 days (n=6), and GTT (l, n=6) and ITT (m, n=4) tests were performed after rmIL-27 treatment for 15 days. A band density was quantified and normalized with ImageJ (a and c), and the experiment was repeated at least twice with similar results. Data was a mean of biologically independent samples ± s.e.m. An unpaired student's *t*-test was shown (e and k); and two-way analysis of variance was shown (d, f, g, j, l and m). **p*<0.05.

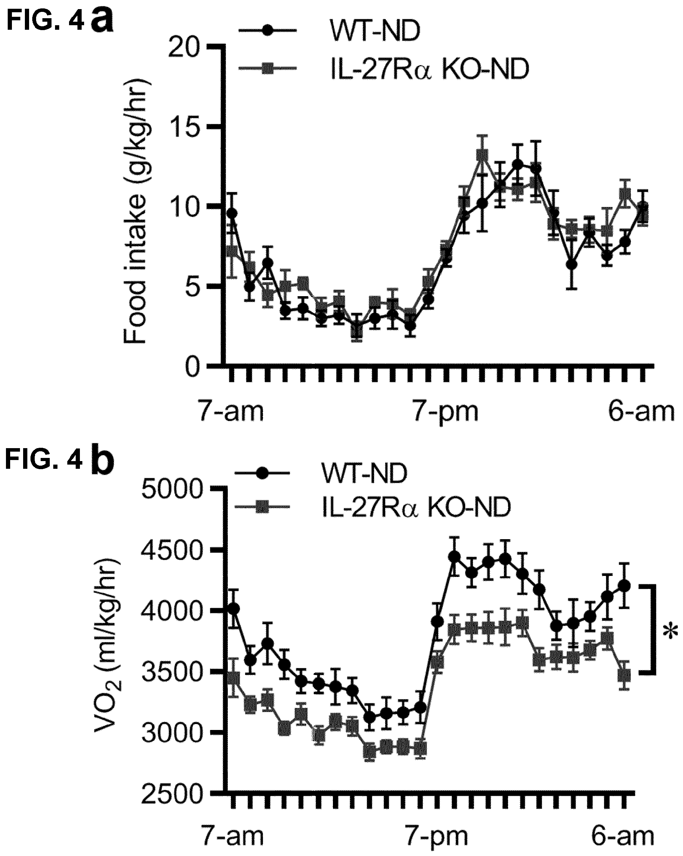

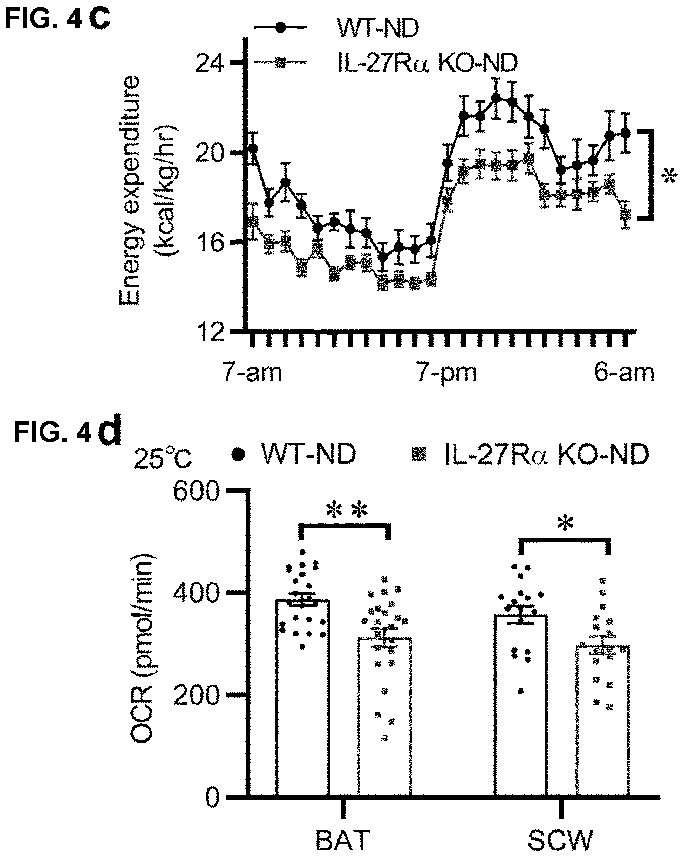

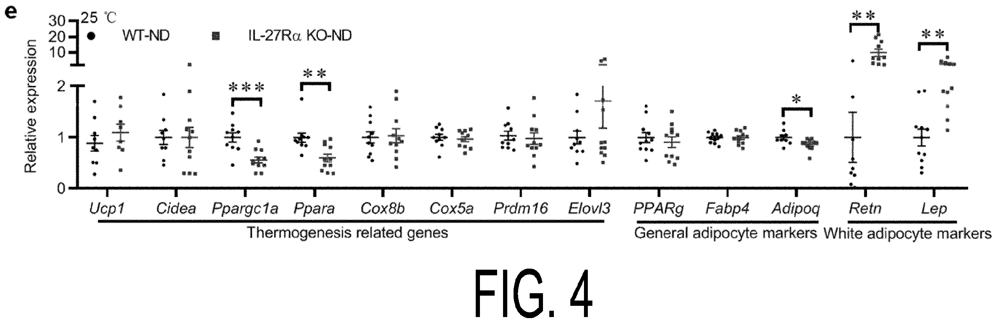

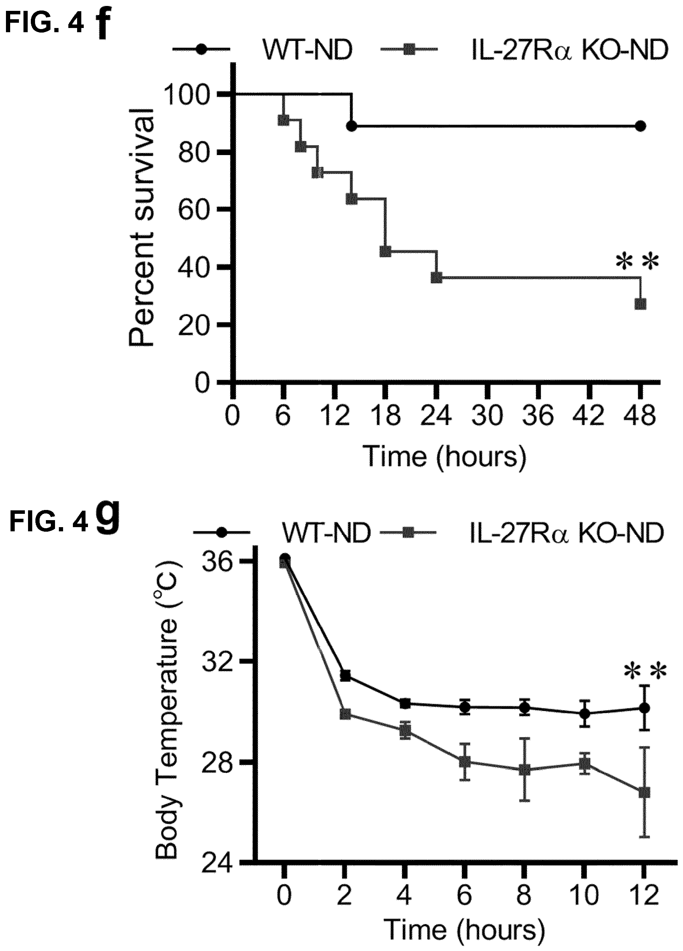

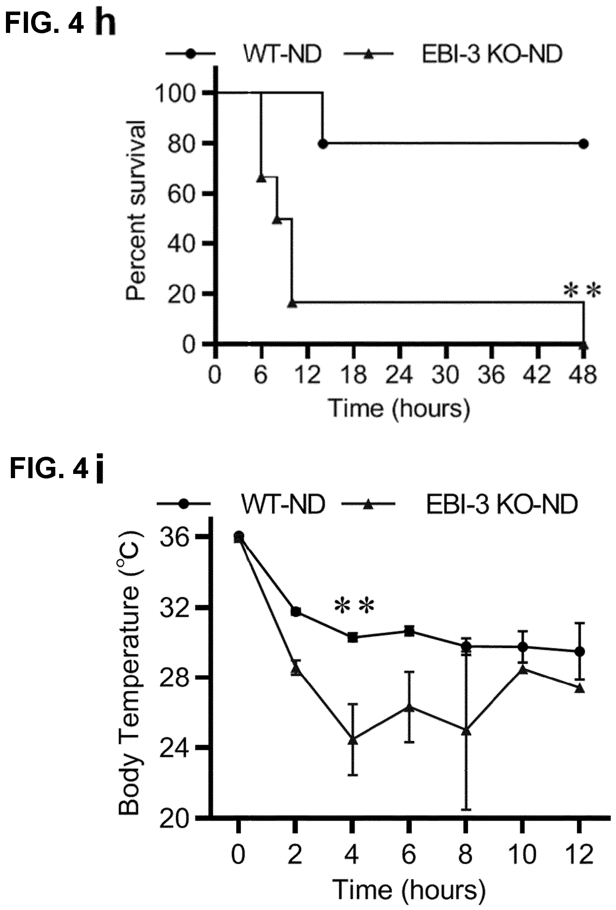

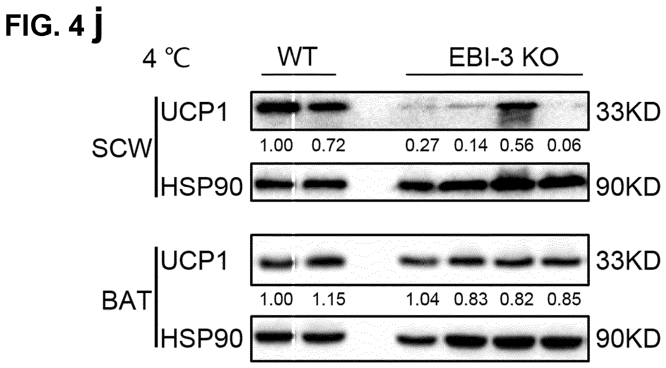

FIG. 4 shows reduction in energy expenditure and heat generation in IL-27 signal-deficient mice. As for a-c: 8-week-old IL-27Rα KO and WT mice were placed in metabolic cages. Food intake (a), oxygen consumption (b) and energy expenditure (c) (n=7-8) were shown. As for d: BAT and SCW tissue was separated from 6-8-week-old WT-ND and IL-27Rα KO and cut into small pieces (about 0.003g BAT and about 0.004g SCW), and a basal oxygen consumption rate (n=5/group, 4-5 pieces/group/mouse) was detected using a Seahorse XF analyzer. As for e: a survival curve of response of IL-27Rα KO and WT mice fed with ND to cold stimulation (4°C, n=9-11) was shown. As for f: an influence of cold stimulation on rectal temperatures of the mice fed with ND (4°C, n=9-11) was shown. As for g: expression of genes of IL-27Rα KO and WT mice fed with a normal diet in SCW was detected using real-time PCR (n=9-11). As for h-j: EBI-3 KO mice and a WT control group fed with a normal diet were recorded at 4°C cold stimulation with survival curves (h) and rectal temperatures (i) (n=5-6) shown in the drawing. BAT and SCW were collected 12 hours after cold stimulation, lysed and used for immunoblot analysis of UCP1 (j). A band density was quantified with ImageJ, and a UCP1/HSP90 ratio was normalized. The experiment was performed twice with similar results. Data was a mean of biologically independent samples ± s.e.m. An unpaired *t*-test (d and g) was shown; two-way analysis of variance (a-c, f and i) was shown; and a log rank test (e and h) was shown. **p*<0.05, ***p*<0.01, and ***p*<0.001.

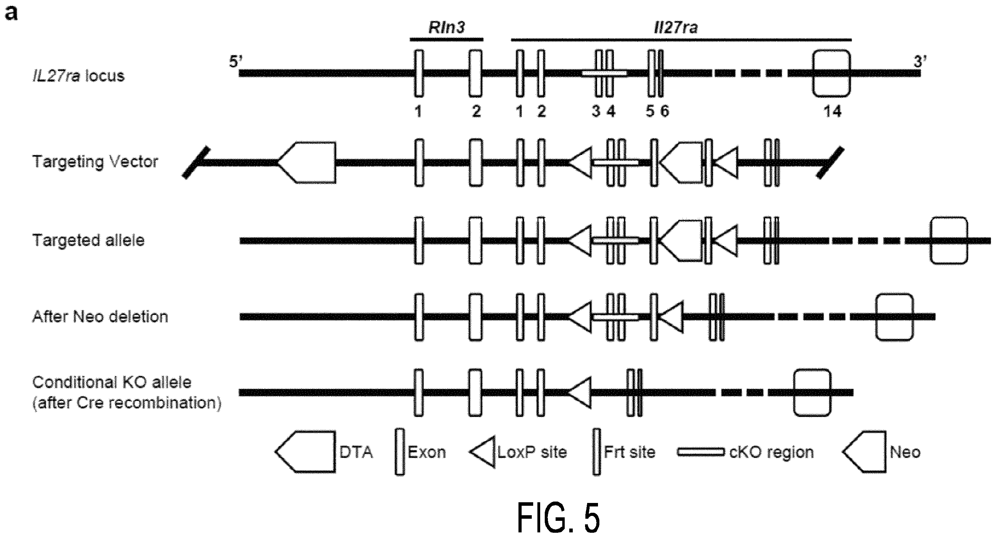

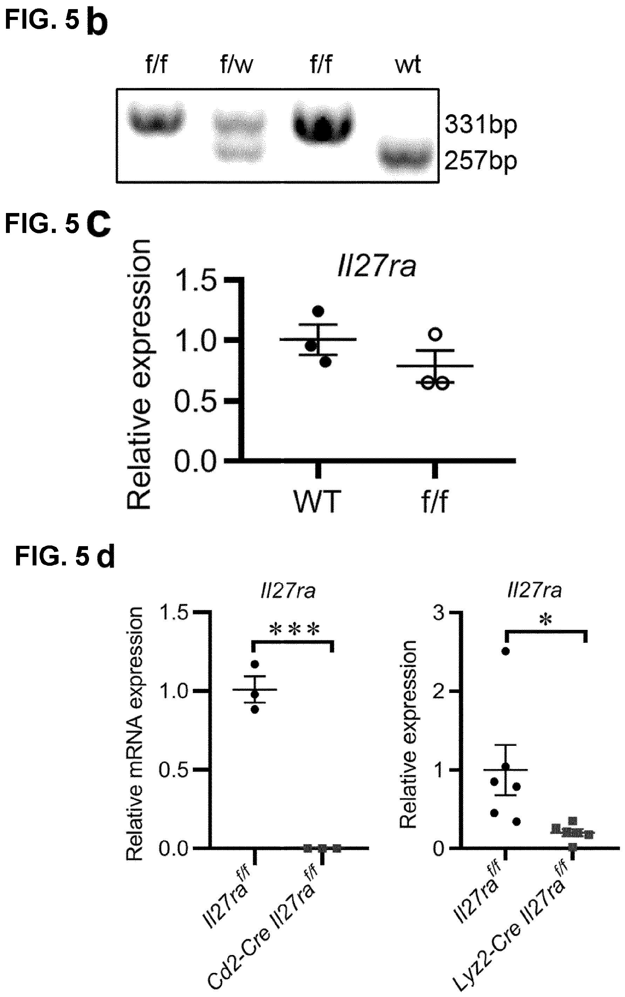

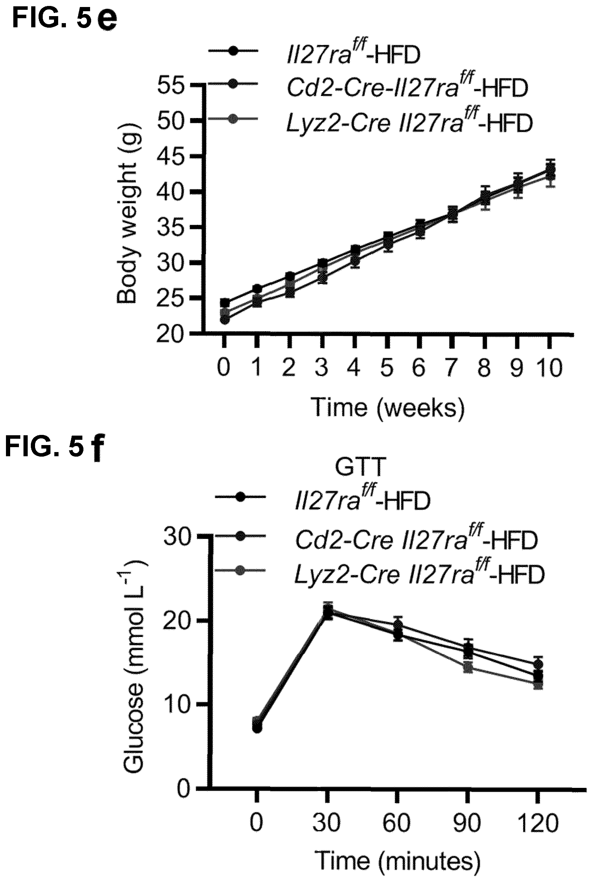

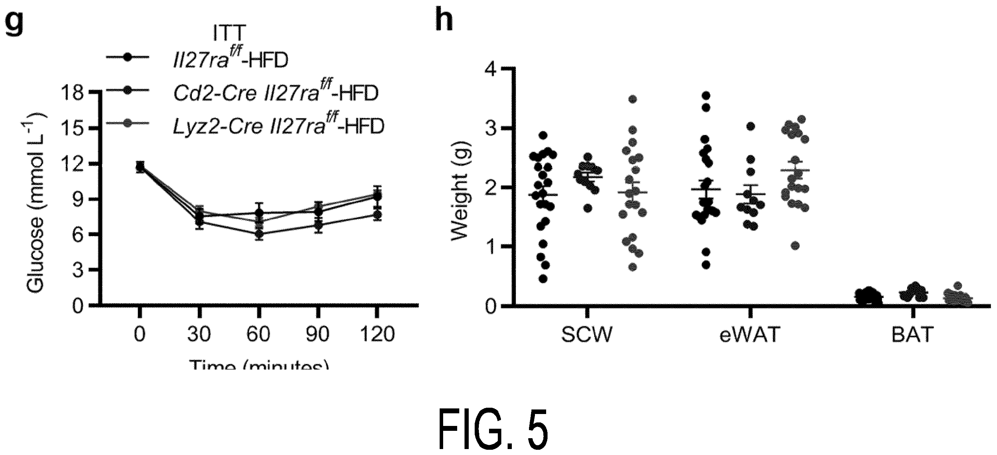

FIG. 5 shows that thermogenesis of IL-27Rα signals was not achieved by direct action on CD2+ lymphocytes or Lyz2+ myeloid cells. As for a: a schematic model generated for *II27ra*^{flox/flox} mice was shown. An *II27ra* site (top) was targeted by a targeting vector (second), and the targeting vector contained a homologous sequences of *II27ra* including two LoxP sites on two sides of exons 3 and 4 and a Neo selection box. The linearized vector was then delivered to embryonic stem cells by electroporation (C57BL/6), followed by drug selection, PCR screening and Southern blot for confirmation. Homologous recombination produced a floxed allele (Third). After correct targeting of ES clones was confirmed by the Southern blot, selected clones were used for blastocyst microinjection to generate generation F0. It was identified that after hybridization of F0 with Flp-deleter, F1 was confirmed as a germ line passage to cancel the Neo box (Forth). After Cre recombination, mismatching with a floxed *II27ra* allele resulted in deletion of exons 3 and 4 (bottom). As for b: genotyping of *II27ra*^{f/f} mice was shown. As for c: expression (n=3) of an *II27ra* gene in spleens of *II27ra*^{f/f} and WT mice was detected by adopting a real-time fluorescent quantitative PCR method. As for d: expression of an *II27ra* gene in CD3 T cells in spleens of *II27ra*^{f/f} and *Cd2-Cre II27ra*^{f/f} mice was detected using real-time fluorescent quantitative PCR (n=3, left. Thioglycollate was injected intraperitoneally into *II27ra*^{f/f} and *Lyz2-Cre II27ra*^{f/f} mice for 4 days, peritoneal macrophages were then collected, and expression of an *II27ra* gene was detected by real-time PCR (n=5-6, right). As for e-h: 8-week-old *II27ra*^{f/f}, Cd2-Cre-II27ra^{f/f} and Lyz2-Cre-II27ra^{f/f} mice were fed with HFD for 10 weeks. As for e: body weight was recorded weekly (n=11-21). GTT (f) and ITT (g) tests (n=11-19) were performed after HFD treatment for 10 weeks. As for h: after HFD treatment for 10 weeks, adipose tissue was collected and weighed (n=11-21). Data was a mean of biologically independent samples ± s.e.m. An unpaired t-test (c, d) was shown; two-way analysis of variance (e-g) was shown; and one-way analysis of variance (h) was shown. **p*<0.05, and ****p*<0.001.

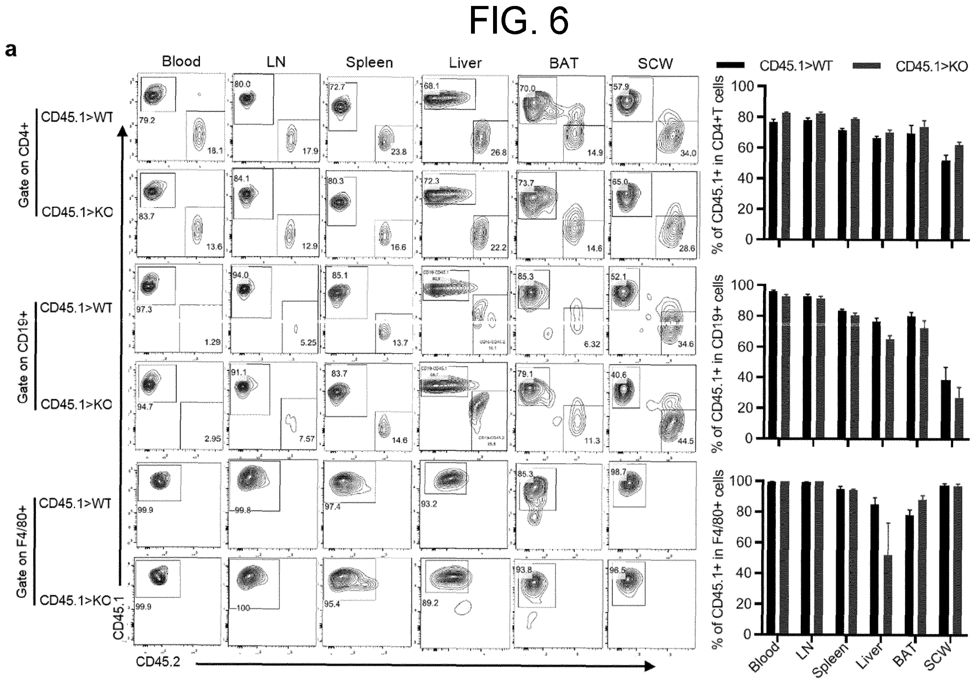

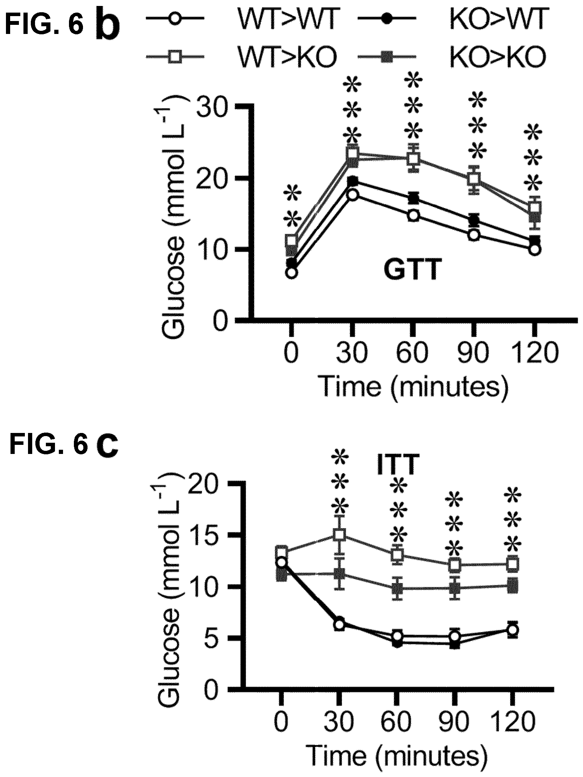

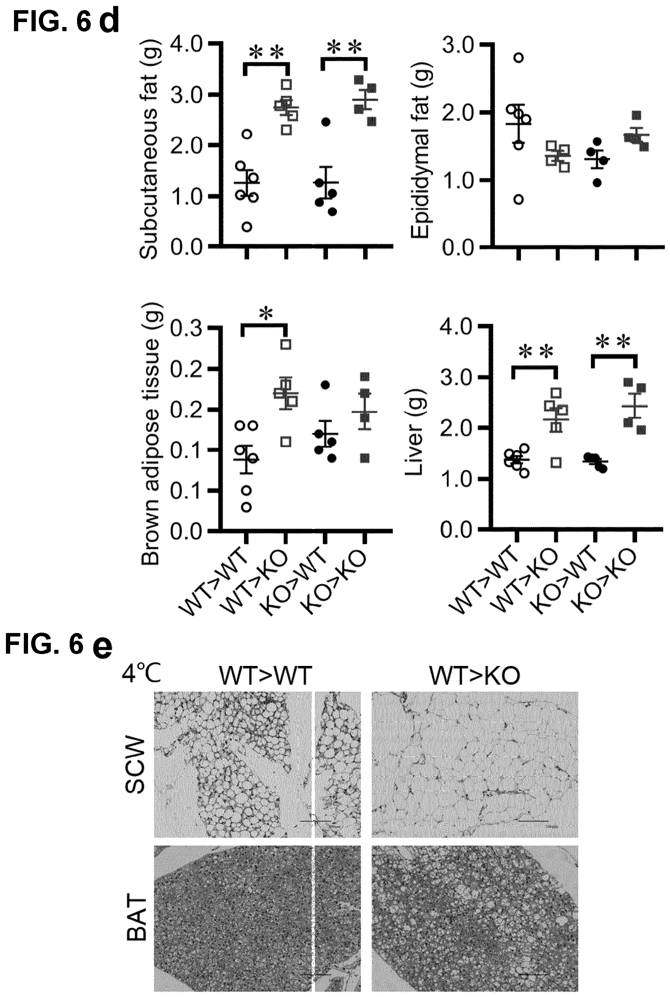

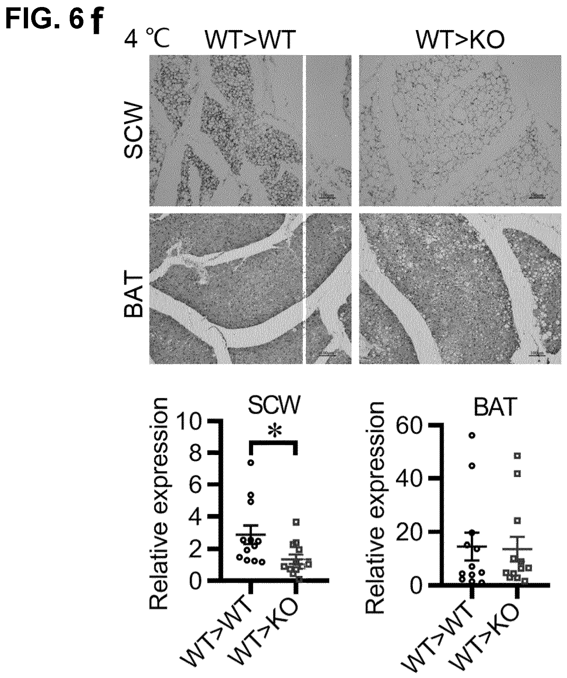

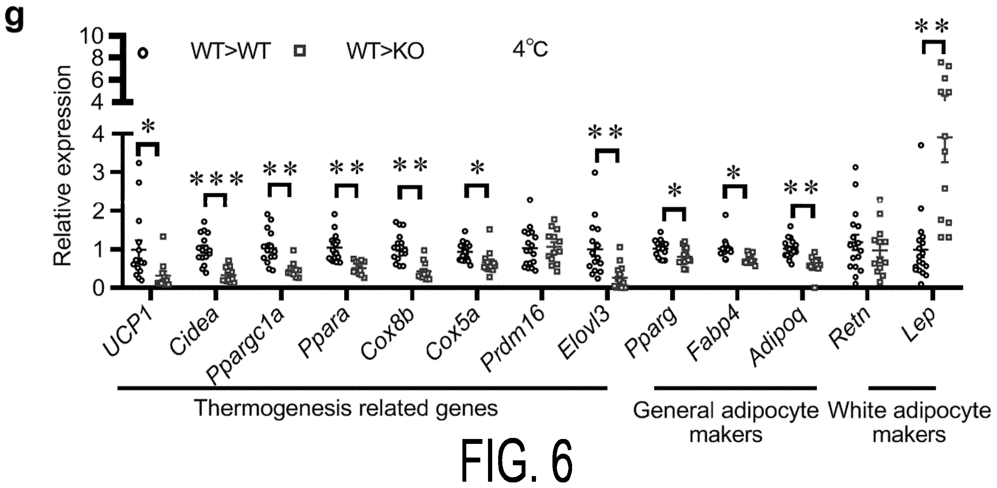

FIG. 6 is a phenotype of an IL-27Rα KO and WT chimera in HFD-induced adaptive heat generation. Bone marrow cells from CD45.1 WT strains were transferred to irradiated CD45.2 IL-27Rα KO or CD45.2 WT hosts (1X10⁷ cells/mouse) to generate chimeras. Mice were housed for 8 weeks to reconstitute immune systems. As shown, immune cells were separated from different tissue, and percentages (n=3) of the immune cells in donor (CD45.1+) and host (CD45.2+) cells in CD4+T (top), CD19+B (middle) or F4/80+ macrophages (bottom) were analyzed by a flow cytometer. As for b-d: bone marrow chimeras were obtained and fed with HFD as shown in a in the drawing. GTT (b, n=10-13) and ITT (c, n=5-7) tests were performed after 10 weeks of HFD treatment. As for d: after 10 weeks of HFD treatment, adipose tissue and livers were collected and weighed (n=4-6). As for e-g: chimera mice fed with a normal diet were subjected to cold stimulation at 4°C for 12 hours, and SCW and BAT were collected for histological analysis (e, H and E, scale=100µm) or UCP1 immunohistochemical staining (f, scale=100µm). Real-time PCR analysis of gene expression of SCW was performed (g, n= 12). Data was a mean of biologically independent samples ± s.e.m. An unpaired t-test (a, f and g) was shown; one-way analysis of variance (d) was shown; and two-way analysis of variance (b and c) was shown. **p*<0.05, ***p*<0.01, and ***p*<0.001.

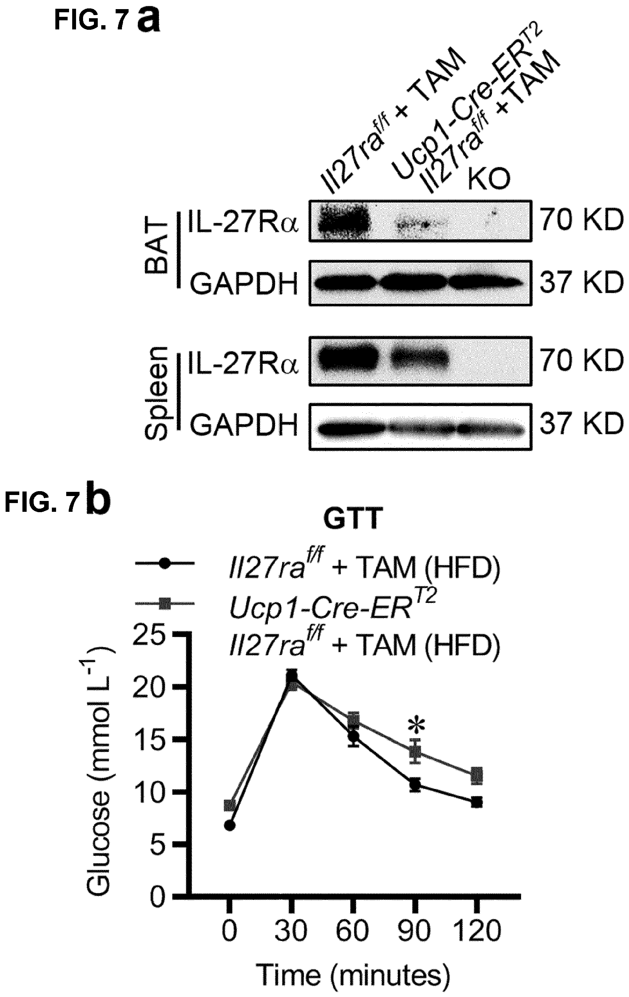

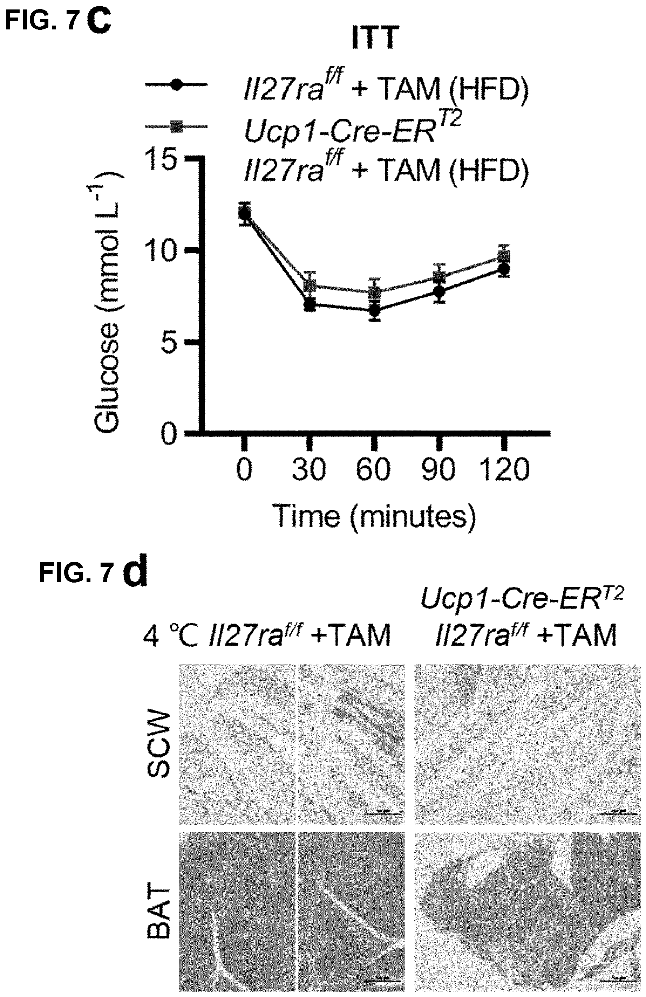

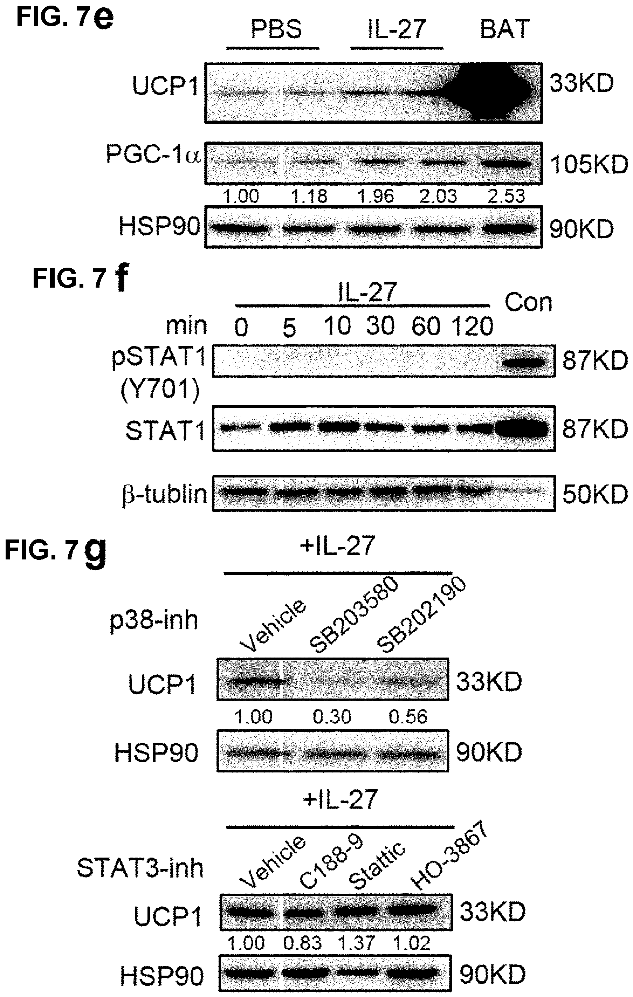

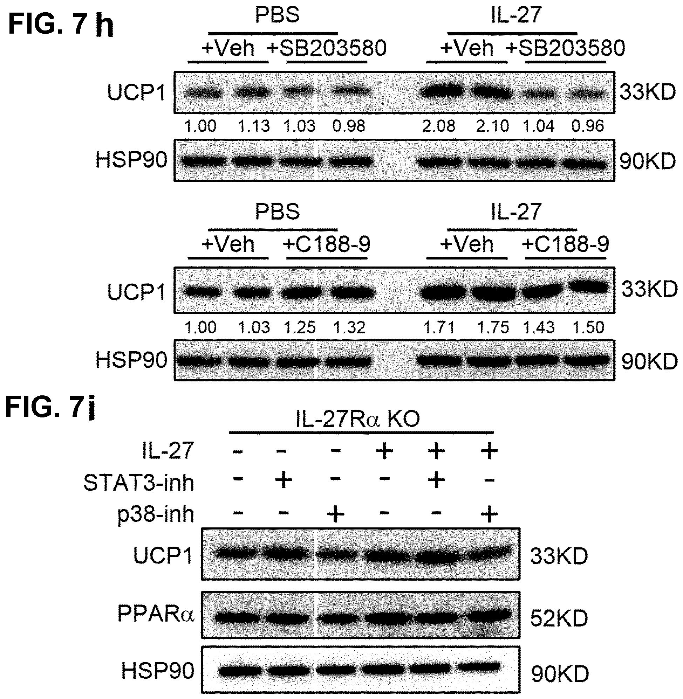

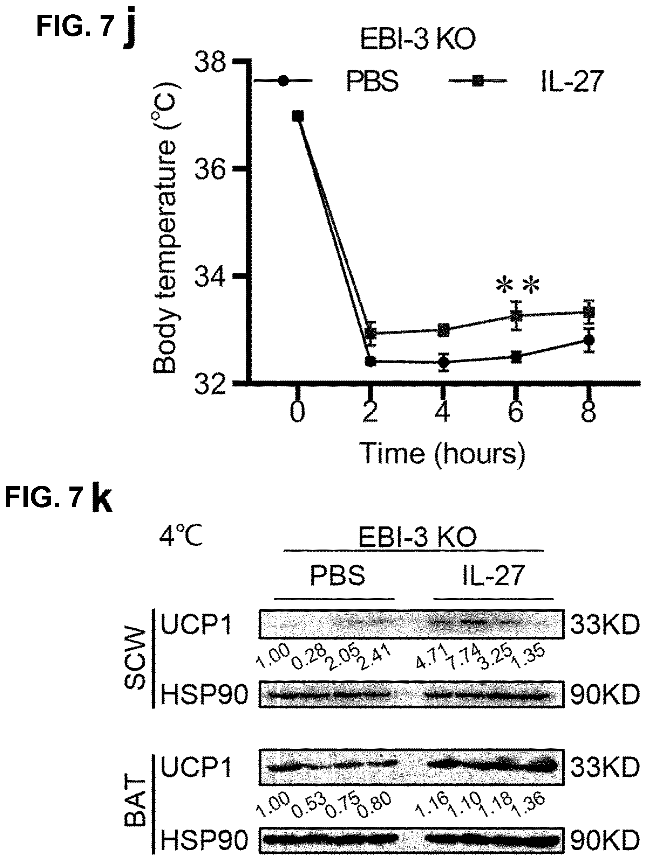

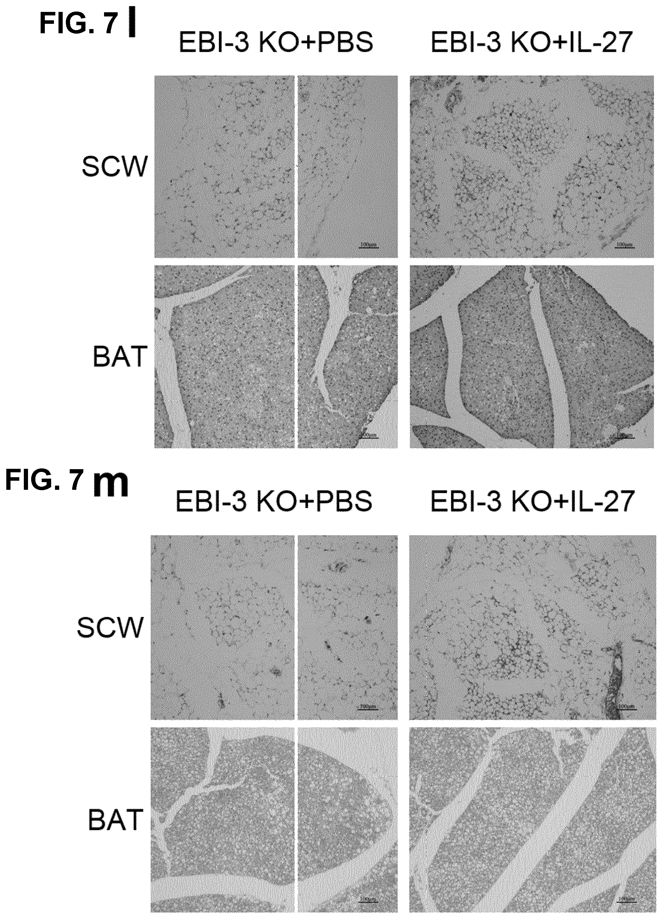

FIG. 7 shows that IL-27 up-regulates UCP1 and improves browning of subcutaneous white adipose tissue. As for a-c: 8-week-old II27ra^{f/f} and *Ucp1-Cre II27ra*^{f/f} mice were fed with HFD for 10 weeks. After HFD treatment for 10 weeks, GTT (a) and ITT (b) (n=5-6) tests were performed, and adipose tissue and livers were collected and weighed (c, n=5-6). As for d: after II27ra^{f/f} and Ucp1-Cre II27ra^{f/f} mice fed with a normal diet were subjected to cold stimulation at 4°C for 12 hours, SCW and BAT were collected for histological analysis (H and E) with the scale=100µm. As for e: primary beige adipocytes were generated in vitro from an SVF of WT SCW and then treated with rmIL-27 (100ng/ml) or PBS for 24 hours. Cells were lysed and a protein extract was used for immunoblot analysis. BAT tissue from WT mice served as positive control. As for f: immunoblot analysis of phosphorylation of STAT1 in the WT primary beige adipocyte extract treated with the rmIL-27 (100ng/ml) for a specified time was shown. As for g: subcutaneous adipocytes of WT mice were cultured in vitro. Two different p38 MAPK inhibitors (SB203580, 10µM and SB202190, 5µM) or three STAT3 inhibitors (C188-9, 10µM; Stattic, 10µM or HO3867, 20µM) were added to a medium 0.5h prior to and during rmIL-27 treatment (100ng/ml, 12h). Expression of UCP1 was detected by immunoblot. As for h-i: primary beige adipocytes of SCW from WT (h) or IL-27Rα KO (i) mice were obtained by in vitro culturing. The p38 MAPK inhibitor (SB203580, 10µM) or STAT3 inhibitor (C188-9, 10µM) was added to a medium 0.5h prior to and during rmIL-27 treatment (100ng/ml, 12h). Expression of UCP1 or PPARα was analyzed by an immunoblot. As for j-m: EBI-3 KO mice were injected intraperitoneally with rmIL-27 (100µg/kg) or PBS for 7 consecutive days, and then, cold stimulation was performed at 4°C. As for j: rectal temperatures of the mice for cold stimulation (n=6) were shown. SCW and BAT protein extracts were used for immunoblot analysis after cold stimulation for 24 hours. UCP1 staining (I) and histological analysis (m) (scale=100µm) were shown. Representative portions were shown. A band density was quantified and normalized with ImageJ. The experiment was repeated twice with similar results. Data was a mean of biologically independent samples ± s.e.m. An unpaired *t-*test (c) was shown; and two-way analysis of variance (a, b and j) was shown. **p*<0.05, ***p*<0.01, and ***p*<0.001.

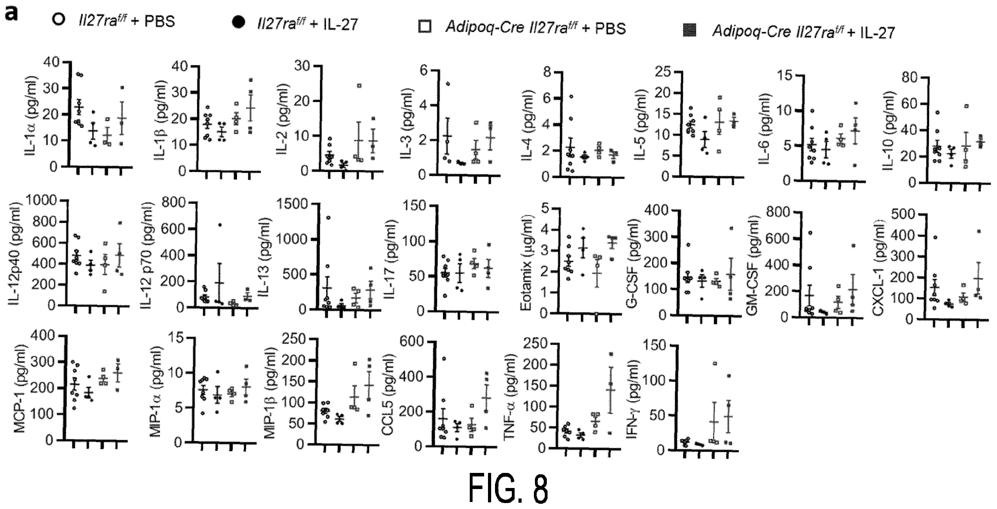

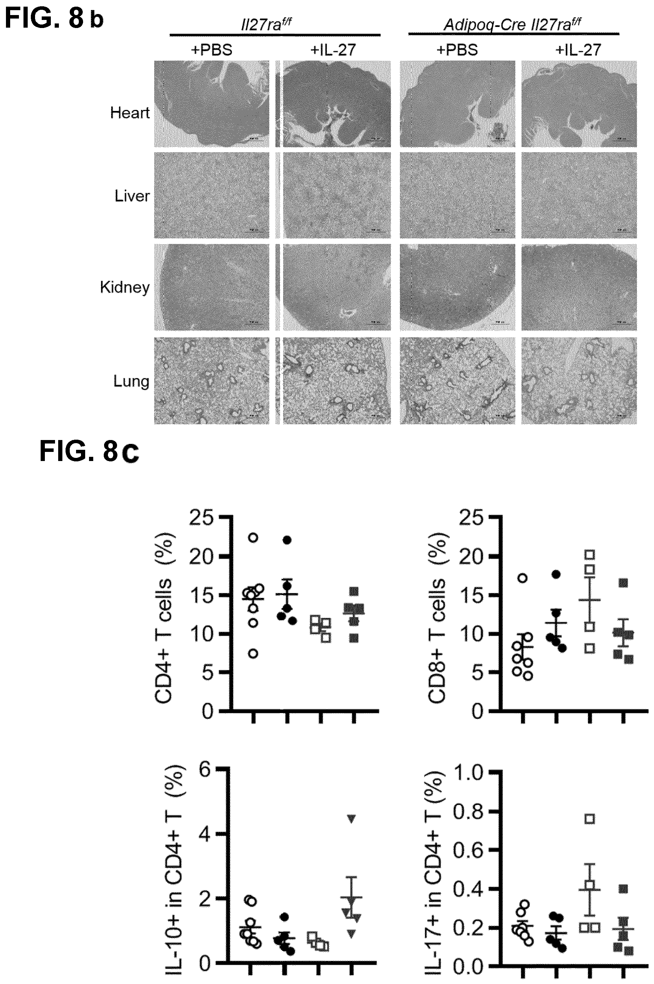

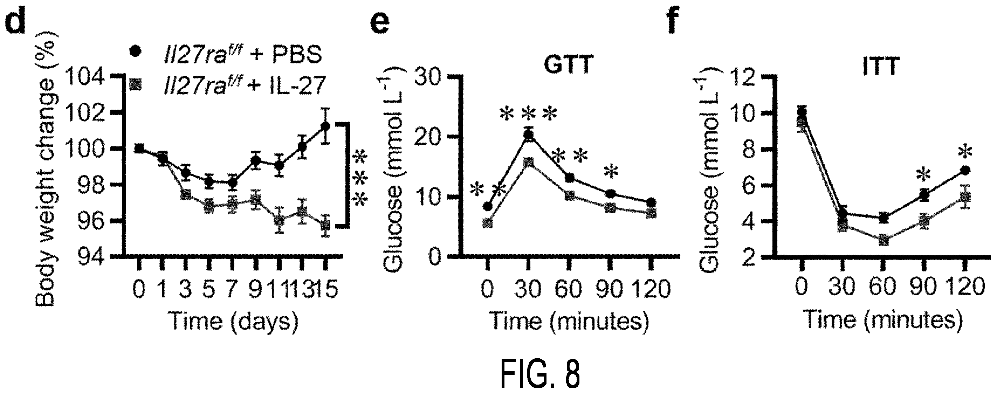

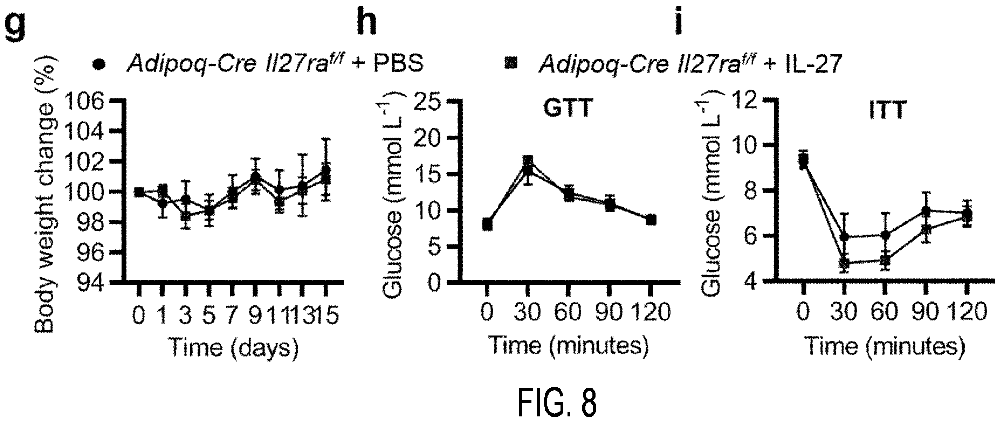

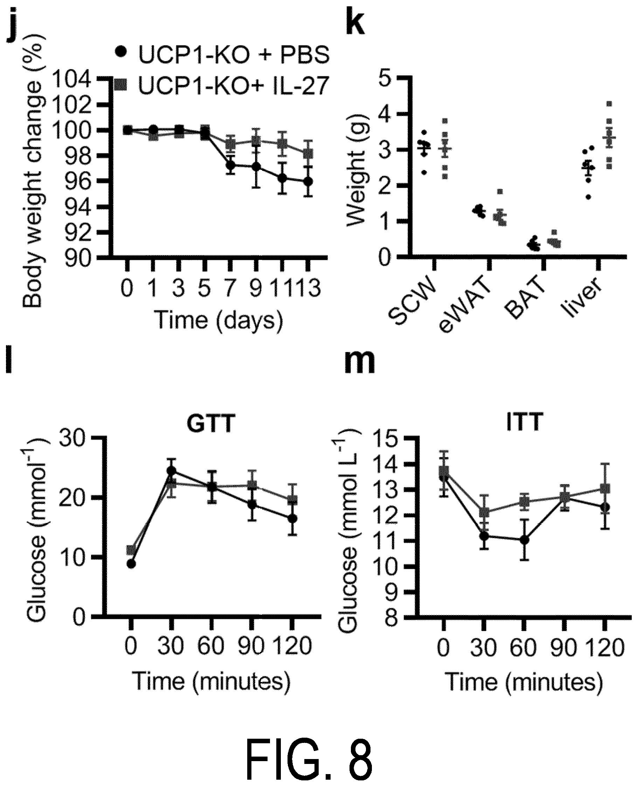

FIG. 8 shows that the effect of IL-27 promoting heat generation. As for a-i: *II27ra*^{f/f} and *Adipoq-Cre II27ra*^{f/f} mice were fed with HFD for 32 weeks, and then, intraperitoneal injection of rmIL-27 (100µg/kg) or PBS was performed every other day for 15 days in total. As for a: inflammatory factors in serum was detected by adopting a Bio-Rad Bio-Plex 200 multifunctional analyzer. As for b: representative tissue sections of indicated tissue (H and E) were shown. As for c: a percentage of infiltrated CD4 or CD8 T cells and production of cytokine of hepatic CD4 T cells were analyzed by a flow cytometer. Body weight (d), GTT (e) and ITT (f) (n=5-8) of *II27ra*^{f/f} mice were detected and recorded. Body weight (g), GTT (h) and ITT (i) (n=4-5) of *Adipoq-Cre II27ra*^{f/f} mice were detected and recorded. As for j-m: *Ucp1-Cre II27ra*^{f/f} mice were fed with HFD for 16 weeks, and then, intraperitoneal injection of rmIL-27 (100µg/kg) or PBS was performed every other day for 15 days in total. Body weight (j), tissue weight (k), a glucose tolerance test (l) and an insulin tolerance test (m) (n=5-6) were measured and recorded. Data was a mean of biologically independent samples ± s.e.m. One-way analysis of variance (a and c) and two-way analysis of variance (d-j, l and m) were shown; and an unpaired *t*-test (k) was shown. **p*<0.05, ***p*<0.01, and ***p*<0.001.

### 4. Conclusion

Browning of white adipose tissue predisposes mice to increase energy expenditure. In order to examine a systemic metabolic status, in the present disclosure, the energy expenditure and nutrient consumption of the IL-27Rα KO mice and the WT control group having the normal diet or HFD treatment were detected. The energy expenditure of the IL-27Rα KO mice fed with the normal diet was significantly reduced without changing the food intake (shown in a-c in FIG. 7). The phenotype was more pronounced when HFD was fed (shown in a-c in FIG. 1). The data reveals that IL-27 signals have an effect of maintaining systemic metabolic homeostasis. To determine whether an influence of IL-27Rα deficiency on the energy expenditure may be caused by reduced heat generation, in the present disclosure, an influence of IL-27 signal absence on adaptive heat generation was then examined.

The IL-27Rα KO mice were sensitive to cold-induced hypothermia under the normal diet condition (shown in f and g in FIG. 4), and this intolerance was more severe after HFD treatment (shown in d and e in FIG. 1). The expression of the key protein (uncoupling protein 1 (UCP1)) regulating thermogenesis in IL-27Rα KO mice was also significantly reduced (shown in f-h in FIG. 1). The tissue sections also showed less browning in the brown adipose tissue (BAT) and subcutaneous white adipose tissue (SCW) in the IL-27Rα KO mice (shown in i in FIG. 1) and fewer cells containing multilumen lipid droplets (shown in j in FIG. 1). The data clearly indicates that the thermogenic activity of the IL-27Rα KO mice is severely impaired. Notably, UCP1 was reduced in the IL-27Rα KO mice prior to cold stimulation (shown in f and h in FIG. 1), as was the case with other thermogenic genes (shown in f in FIG. 4), which was consistent with reduction in systemic energy expenditure (shown in b and c in FIG. 1, and b and c in FIG. 4). Furthermore, cold stimulation for the EBI-3 KO mice reproduced the findings in the IL-27Rα KO mice (shown in g-i in FIG. 4), which further confirms the thermogenic effect of the IL-27 signals.

Next, the cell types responsible for IL-27 mediated signaling were dissected. First, 4 groups of chimera mice (WT>WT, WT>KO, KO>WT and KO>KO) were generated from the bone marrow of the IL-27Rα KO or WT mice, and these mice were subjected to HFD treatment. Surprisingly, only the chimeras made with IL-27Rα KO (no matter what the donor cells were) were highly susceptible to HFD-induced fat accumulation and cold stimulation (shown in a-c in FIG. 2). Compared to wild-type chimeras, thermogenesis of the IL-27Rα KO chimeras was consistently inhibited, which was manifested by a decreased body temperature, decreased multilumen lipid droplets and decreased UCP1 production (shown in a-c in FIG. 2). The study indicates that a dominant target of IL-27 is adipocytes instead of immune cells. In fact, IL-27Rα was expressed on mature adipocytes and primary beige adipocytes. Furthermore, heat generation in IL-27Rα^{△adipo} mice was also inhibited by intolerance to cold stimulation, reduced browning, and reduced UCP1 production (shown in c, e, f and h in FIG. 2). In conclusion, the above data indicates that IL-27 promotes heat generation by directly targeting adipocytes.

To investigate the molecular mechanism of action of IL-27 on adipocytes, primary beige adipocytes were directly treated with IL-27 in vitro. The expression of UCP1 was significantly increased after IL-27 treatment, and meanwhile, the expression of the peroxisome proliferator-activated receptor α (PPARα), the peroxisome proliferator-activated receptor γ co-activator 1α (PGC-1α) and the key transcriptional co-activator mediating energy metabolism was up-regulated (shown in a in FIG. 3). The typical downstream signal pathways for IL-27Rα in immune cells were JAK/STAT3 and p38 MAPK. Next, whether IL-27 promoted heat generation of the adipocytes by these pathways was examined. Phosphorylation of p38 MAPK was significantly increased under IL-27 stimulation, whereas pSTAT3 was only transiently induced and rapidly regressed (shown in b in FIG. 3). It was known that p38 MAPK activates ATF2-mediated transcription of PGC-1α, and PGC-1α is a major regulator of UCP1 and thermogenesis. As expected, IL-27 treatment also enhanced the activation of ATF2 in the primary beige adipocytes, and then, PGC-1α was up-regulated, which could be canceled by pharmacological inhibition of the activity of p38 MAPK (shown in a-c in FIG. 3). Furthermore, cells with p38 MAPK inhibition were tolerant to IL-27-induced UCP1 promotion (shown in c in FIG. 3). However, the response of up regulation of UCP1 by IL-27 to STAT3 inhibition was essentially unaffected, although it also decreased the expression of PGC-1α (shown in c in FIG. 3). These results indicates that IL-27 enhances thermogenesis mainly through the p38 MAPK-PGC-1α signal pathway. Interestingly, inhibition of both p38 and STAT3 failed to cancel the up regulation of PPARα, which suggests that effects of IL-27 may involve additional pathways.

Furthermore, supplementation of the EBI-3 KO mice with IL-27 improved UCP1-mediated heat generation and avoided low temperatures in cold stimulation (FIG. 4).

In summary, the results indicate that the IL-27 signal is indispensable for the full adaptive heat generation. Direct targeting of IL-27 to adipocytes induces activation of p38 MAPK, thereby driving activation of ATF2 and subsequent expression of PGC-1α and UCP1. In conclusion, these findings provides new insights and extends understanding of the mechanism of in vivo heat generation.

### Industrial applicability

The present disclosure provides the composition for controlling heat generation within an organism, and the use, and the present disclosure discloses the functions of the IL-27 signals in adjustment of heat generation. Molecular mechanism research proves that the IL-27 signals directly act on adipocytes, activate p38 MAPK-PGC1 signals and stimulate generation of UCP1. Therapeutic administration of IL-27 reverses hypothermia of EBI-3 KO mice induced at the low temperature. Therefore, the present disclosure reveals a key role of the IL-27 signals in an energy metabolism process, provides a feasible solution for controlling the body temperature, and has a remarkable industrial practical performance and high market value.

## Claims

1. A molecule for a therapeutic use in reducing or increasing a body temperature of an organism in need thereof, **characterized in that**
the molecule for use in reducing the body temperature of the organism is a molecule inhibiting or reducing the binding of EBI-3 and p28 or a molecule inhibiting or reducing the binding of dimers of EBI-3 and p28 and receptors thereof, and
the molecule for use in increasing the body temperature of the organism is a molecule promoting or enhancing the binding of EBI-3 and p28 or a molecule promoting or enhancing the binding of dimers of EBI-3 and p28 and receptors thereof.

2. The molecule for use according to claim 1, wherein the molecule for use in reducing the body temperature of the organism comprises a biological macromolecule or small molecule compound, comprising an anti-EBI-3 antibody, an anti-p28 antibody, an anti-IL-27R antibody, an EBI-3 soluble fragment, a p28 soluble fragment and an IL-27R soluble fragment.

3. The molecule for use according to claim 1, wherein the molecule for use in increasing the body temperature of the organism is a recombinant IL-27 protein.

4. A in vitro method for screening a candidate compound capable of reducing or increasing a body temperature of an organism, **characterized by** comprising a step of measuring a binding condition of EBI-3 and p28, or a binding condition of dimers of EBI-3 and p28 and receptors thereof, before and after application of a to-be-tested compound,
wherein when the binding of EBI-3 and p28 is inhibited or reduced or the binding of the dimers of EBI-3 and p28 and the receptors thereof is inhibited or reduced after the to-be-tested compound is applied, the to-be-tested compound is identified as a candidate compound for reducing the body temperature of the organism,
wherein when the binding of EBI-3 and p28 is promoted or enhanced or the binding of the dimers of EBI-3 and p28 and the receptors thereof is promoted or enhanced after the to-be-tested compound is applied, the to-be-tested compound is identified as a candidate compound for increasing the body temperature of the organism.

5. The method according to claim 4, comprising:
(1) measuring a parameter of the binding of EBI-3 and p28 or a parameter of the binding of the dimers of EBI-3 and p28 and the receptors thereof, in an organism model, so as to obtain a first measured value;
(2) after the to-be-tested compound is applied to the organism model, measuring the parameter of the binding of EBI-3 and p28 or the parameter of the binding of the dimers of EBI-3 and p28 and the receptors thereof, in the organism model, so as to obtain a second measured value; and
(3) comparing the first measured value and the second measured value.

6. The method according to claim 4, comprising:
(1) measuring a parameter of the binding of EBI-3 and p28 or a parameter of the binding of the dimers of EBI-3 and p28 and the receptors thereof, in a first organism model, so as to obtain a first measured value;
(2) measuring a parameter of the binding of EBI-3 and p28 or a parameter of the binding of the dimers of EBI-3 and p28 and the receptors thereof, in a second organism model, so as to obtain a second measured value, wherein the first organism model and the second organism model belong to the same model, the to-be-tested compound is not applied to the first organism model, and the to-be-tested compound is applied to the second organism model; and
(3) comparing the first measured value and the second measured value.

7. The method according to claim 4, comprising:
(1) measuring a parameter of the binding of EBI-3 and p28 or a parameter of the binding of the dimers of EBI-3 and p28 and the receptors thereof, in an organism model after the to-be-tested compound is applied, so as to obtain a measured value; and
(2) comparing the measured value with a reference value, wherein the reference value is a parameter of the binding of EBI-3 and p28 or a parameter of the binding of the dimers of EBI-3 and p28 and the receptors thereof, measured by the organism model when the to-be-tested compound is not applied.

8. The method according to any one of claims 5 to 7, wherein the organism model comprises an animal model or a cell model.

## Patentansprüche

1. Molekül zur therapeutischen Verwendung bei der Senkung oder Erhöhung einer Körpertemperatur eines Organismus, der dieser bedarf, **dadurch gekennzeichnet, dass**
das Molekül zur Verwendung bei der Senkung der Körpertemperatur des Organismus ein Molekül ist, das die Bindung von EBI-3 und p28 hemmt oder verringert, oder ein Molekül ist, das die Bindung von Dimeren von EBI-3 und p28 und Rezeptoren davon hemmt oder verringert, und
das Molekül zur Verwendung bei der Erhöhung der Körpertemperatur des Organismus ein Molekül ist, das die Bindung von EBI-3 und p28 fördert oder verstärkt, oder ein Molekül ist, das die Bindung von Dimeren von EBI-3 und p28 und Rezeptoren davon fördert oder verstärkt.

2. Molekül zur Verwendung nach Anspruch 1, wobei das Molekül zur Verwendung bei der Senkung der Körpertemperatur des Organismus ein biologisches Makromolekül oder eine Kleinmolekülverbindung ist, umfassend einen Anti-EBI-3-Antikörper, einen Anti-p28-Antikörper, einen Anti-IL-27R-Antikörper, ein EBI-3-lösliches Fragment, ein p28-lösliches Fragment und ein IL-27R-lösliches Fragment.

3. Molekül zur Verwendung nach Anspruch 1, wobei das Molekül zur Verwendung bei der Erhöhung der Körpertemperatur des Organismus ein rekombinantes IL-27-Protein ist.

4. In-vitro-Verfahren zum Screenen einer Kandidatenverbindung, die dazu in der Lage ist, eine Körpertemperatur eines Organismus zu senken oder zu erhöhen, **gekennzeichnet durch** Umfassen eines Schritts eines Messens eines Bindungszustands von EBI-3 und p28 oder eines Bindungszustands von Dimeren von EBI-3 und p28 und Rezeptoren davon vor und nach Anwendung einer zu testenden Verbindung,
wobei, wenn die Bindung von EBI-3 und p28 gehemmt oder verringert ist oder die Bindung der Dimere von EBI-3 und p28 und der Rezeptoren davon gehemmt oder verringert ist, nachdem die zu testende Verbindung angewendet wurde, die zu testende Verbindung als eine Kandidatenverbindung zum Senken der Körpertemperatur des Organismus identifiziert wird,
wobei, wenn die Bindung von EBI-3 und p28 gefördert oder verstärkt ist oder die Bindung der Dimere von EBI-3 und p28 und der Rezeptoren davon gefördert oder verstärkt ist, nachdem die zu testende Verbindung angewendet wurde, die zu testende Verbindung als eine Kandidatenverbindung zum Erhöhen der Körpertemperatur des Organismus identifiziert wird.

5. Verfahren nach Anspruch 4, umfassend:
(1) Messen eines Parameters der Bindung von EBI-3 und p28 oder eines Parameters der Bindung der Dimere von EBI-3 und p28 und der Rezeptoren davon in einem Organismusmodell, um einen ersten Messwert zu erhalten;
(2) nachdem die zu testende Verbindung auf das Organismusmodell angewendet wurde, Messen des Parameters der Bindung von EBI-3 und p28 oder des Parameters der Bindung der Dimere von EBI-3 und p28 und der Rezeptoren davon in dem Organismusmodell, um einen zweiten Messwert zu erhalten; und
(3) Vergleichen des ersten Messwerts und des zweiten Messwerts.

6. Verfahren nach Anspruch 4, umfassend:
(1) Messen eines Parameters der Bindung von EBI-3 und p28 oder eines Parameters der Bindung der Dimere von EBI-3 und p28 und der Rezeptoren davon in einem ersten Organismusmodell, um einen ersten Messwert zu erhalten;
(2) Messen eines Parameters der Bindung von EBI-3 und p28 oder eines Parameters der Bindung der Dimere von EBI-3 und p28 und der Rezeptoren davon in einem zweiten Organismusmodell, um einen zweiten Messwert zu erhalten, wobei das erste Organismusmodell und das zweite Organismusmodell zu demselben Modell gehören, wobei die zu testende Verbindung nicht auf das erste Organismusmodell angewendet wird und die zu testende Verbindung auf das zweite Organismusmodell angewendet wird; und
(3) Vergleichen des ersten Messwerts und des zweiten Messwerts.

7. Verfahren nach Anspruch 4, umfassend:
(1) Messen eines Parameters der Bindung von EBI-3 und p28 oder eines Parameters der Bindung der Dimere von EBI-3 und p28 und der Rezeptoren davon in einem Organismusmodell, nachdem die zu testende Verbindung angewendet wurde, um einen Messwert zu erhalten;
(2) Vergleichen des Messwerts mit einem Referenzwert, wobei der Referenzwert ein Parameter der Bindung von EBI-3 und p28 oder ein Parameter der Bindung der Dimere von EBI-3 und p28 und der Rezeptoren davon ist, gemessen durch das Organismusmodell, wenn die zu testende Verbindung nicht angewendet wird.

8. Verfahren nach einem der Ansprüche 5 bis 7, wobei das Organismusmodell ein Tiermodell oder ein Zellmodell umfasst.

## Revendications

1. Molécule pour une utilisation thérapeutique dans la réduction ou l'augmentation d'une température corporelle d'un organisme en ayant besoin, **caractérisée en ce que**
la molécule pour une utilisation dans la réduction de la température corporelle de l'organisme est une molécule inhibant ou réduisant la liaison d'EBI-3 et de p28 ou une molécule inhibant ou réduisant la liaison de dimères d'EBI-3 et de p28 et de leurs récepteurs, et
la molécule pour une utilisation dans l'augmentation de la température corporelle de l'organisme est une molécule favorisant ou améliorant la liaison d'EBI-3 et de p28 ou une molécule favorisant ou améliorant la liaison de dimères d'EBI-3 et de p28 et de leurs récepteurs.

2. Molécule pour une utilisation selon la revendication 1, laquelle molécule pour une utilisation dans la réduction de la température corporelle de l'organisme comprend une macromolécule biologique ou un petit composé moléculaire, comprenant un anticorps anti-EBI-3, un anticorps anti-p28, un anticorps anti-IL-27R, un fragment soluble d'EBI-3, un fragment soluble de p28 et un fragment soluble d'IL-27R.

3. Molécule pour une utilisation selon la revendication 1, laquelle molécule pour une utilisation dans l'augmentation de la température corporelle de l'organisme est une protéine IL-27 recombinante.

4. Procédé in vitro pour sélectionner un composé candidat capable de réduire ou d'augmenter une température corporelle d'un organisme, **caractérisé en ce qu'**il comprend une étape de mesure d'une condition de liaison d'EBI-3 et de p28, ou une condition de liaison de dimères d'EBI-3 et de p28 et de leurs récepteurs, avant et après application d'un composé à tester,
dans lequel lorsque la liaison d'EBI-3 et de p28 est inhibée ou réduite ou la liaison des dimères d'EBI-3 et de p28 et de leurs récepteurs est inhibée ou réduite après application du composé à tester, le composé à tester est identifié comme composé candidat pour réduire la température corporelle de l'organisme,
dans lequel lorsque la liaison d'EBI-3 et de p28 est favorisée ou améliorée ou la liaison des dimères d'EBI-3 et de p28 et de leurs récepteurs est favorisée ou améliorée après application du composé à tester, le composé à tester est identifié comme composé candidat pour augmenter la température corporelle de l'organisme.

5. Procédé selon la revendication 4, comprenant :
(1) la mesure d'un paramètre de la liaison d'EBI-3 et de p28 ou d'un paramètre de la liaison des dimères d'EBI-3 et de p28 et de leurs récepteurs, dans un modèle d'organisme, de façon à obtenir une première valeur mesurée ;
(2) après application du composé à tester sur le modèle d'organisme, la mesure du paramètre de la liaison d'EBI-3 et de p28 ou du paramètre de la liaison des dimères d'EBI-3 et de p28 et de leurs récepteurs, dans le modèle d'organisme, de façon à obtenir une seconde valeur mesurée ; et
(3) la comparaison de la première valeur mesurée et de la seconde valeur mesurée.

6. Procédé selon la revendication 4, comprenant :
(1) la mesure d'un paramètre de la liaison d'EBI-3 et de p28 ou d'un paramètre de la liaison des dimères d'EBI-3 et de p28 et de leurs récepteurs, dans un premier modèle d'organisme, de façon à obtenir une première valeur mesurée ;
(2) la mesure d'un paramètre de la liaison d'EBI-3 et de p28 ou d'un paramètre de la liaison des dimères d'EBI-3 et de p28 et de leurs récepteurs, dans un second modèle d'organisme, de façon à obtenir une seconde valeur mesurée, dans lequel le premier modèle d'organisme et le second modèle d'organisme appartiennent au même modèle, le composé à tester n'est pas appliqué sur le premier modèle d'organisme et le composé à tester est appliqué sur le second modèle d'organisme ; et
(3) la comparaison de la première valeur mesurée et de la seconde valeur mesurée.

7. Procédé selon la revendication 4, comprenant :
(1) la mesure d'un paramètre de la liaison d'EBI-3 et de p28 ou d'un paramètre de la liaison des dimères d'EBI-3 et de p28 et de leurs récepteurs, dans un modèle d'organisme après application du composé à tester, de façon à obtenir une valeur mesurée ; et
(2) la comparaison de la valeur mesurée avec une valeur de référence, dans lequel la valeur de référence est un paramètre de la liaison d'EBI-3 et de p28 ou d'un paramètre de la liaison des dimères d'EBI-3 et de p28 et de leurs récepteurs, mesuré par le modèle d'organisme lorsque le composé à tester n'est pas appliqué.

8. Procédé selon l'une quelconque des revendications 5 à 7, dans lequel le modèle d'organisme comprend un modèle d'animal ou un modèle de cellule.
